# EUROPEAN PATENT APPLICATION

(11) **EP 4 083 059 A1**
(43) Date of publication of application: **02.11.2022**
(21) Application number: 20893928.0
(22) Date of filing: 24.11.2020
(51) Int. Cl.: C07K 14/705, C12N 7/00, C12N 1/21, C12N 15/12, C12N 15/63, C12P 21/00

(54) **IMPROVED ADENO-ASSOCIATED VIRUS-BINDING PROTEIN, METHOD FOR PRODUCING SAME, AND ADENO-ASSOCIATED VIRUS ADSORBENT USING SAME**

(30) Priority: 25.11.2019 JP 2019212020; 30.09.2020 JP 2020165229
(71) Applicant: Tosoh Corporation, Yamaguchi 746-8501 (JP)
(72) Inventor: ASAOKA, Yoshiharu, Ayase-shi, Kanagawa 252-1123 (JP); KOBAYASHI, Hidetaka, Ayase-shi, Kanagawa 252-1123 (JP); MAKINO, Yuriko, Ayase-shi, Kanagawa 252-1123 (JP); YOSHIDA, Kouhei, Ayase-shi, Kanagawa 252-1123 (JP); KURIHARA, Kento, Ayase-shi, Kanagawa 252-1123 (JP)
(74) Representative: Novagraaf International SA
(86) International application number: PCT/JP2020/043691
(87) International publication number: WO 2021/106882

(57) **Abstract**

The present invention provides an improved adeno-associated virus (AAV)-binding protein having enhanced stability, especially , heat stability, acid stability and alkali stability, of an AAV-binding protein, a method for producing the improved AAV-binding protein, and an AAV adsorbent using the same.

## Description

### FIELD

The present invention relates to a protein having adeno-associated virus (AAV)-binding ability. More specifically, the invention relates to an improved AAV-binding protein having higher stability to heat, acid, and alkali than the wild type, a method for producing the protein, and an AAV adsorbent using the protein.

### BACKGROUND

AAV is a non-enveloped virus classified into the genus *Dependoparvovirus* of the family *Parvoviridae.* AAV capsid particles are composed of about 60 protein molecules in a ratio of 1:1:10 of three types of proteins (VP1, VP2, and VP3), and have the shape of a regular icosahedron with a diameter of from 20 to 30 nm.

AAV in nature lacks autonomous proliferation capacity and replication depends on helper viruses such as adenoviruses and herpesviruses. In the presence of the helper viruses, the AAV genome is replicated within host cells to form complete AAV particles containing the AAV genome, and the AAV particles are released from the host cells. On the other hand, in the absence of the helper viruses, the AAV genome is in a state of being maintained in the episome or integrated into the host chromosome (latent state).

AAV can infect cells of a wide variety of species, including human, and also infect non-dividing cells that have been differentiated such as blood cells, muscles, and nerve cells, AAV is less concerned about side effects because it is not pathogenic to humans, AAV particles are physicochemically stable, and the like. Therefore, AAV is attracting attention for its utility value as a vector for gene transfer for the treatment of congenital genetic diseases.

In general, production of recombinant AAV vector (hereinafter, also simply referred to as "AAV vector") is carried out by introducing nucleic acids that encode essential elements for AAV particle formation into cells to prepare cells capable of producing AAV (hereinafter also referred to as "AAV-producing cells") and culturing the cells to express the essential elements for AAV particle formation. AAV is usually produced by introducing the following three types of plasmids into cells: a plasmid (a vector plasmid) for supplying recombinant AAV genome carrying a polynucleotide encoding proteins to be expressed and having inverted terminal repeat (ITR) sequences at both ends; a plasmid (a packaging plasmid) for supplying Rep protein and capsid proteins (outer shell constituent proteins VP1, VP2, and VP3); and a plasmid (a helper plasmid) for supplying only the elements essential for AAV particle formation among the adenovirus-derived elements. As another method, a method using a cell in which elements essential for AAV particle formation are introduced into host cells in advance or a method using insect cells (Sf9 cells or the like) is also used for AAV production.

In gene transfer by an AAV vector, the AAV vector needs to enter into a cell. It is known that AAV binds to the cell surface and receptors at the first step of entry into the cell. This binding involves heparan sulfate, integrin, fibroblast growth factors (FGF) receptor, hepatocyte growth factor (HGF) receptor, and the like. It is generally known that the receptor to which the AAV vector binds when entering the cell differs depending on the serotype of the AAV vector.

Further, a receptorcalled KIAA0319L (AAVR) has been reported as a universal receptor (NPL 1). AAVR is composed of five extracellular domains, a transmembrane domain, and an intracellular domain, and it has been reported that domain 2 (PKD2), which is an extracellular domain, is important for binding to AAV (NPL 2). Since AAVR can bind to an AAV vector, it can be a ligand for adsorbing AAVs. However, since AAVR is a human-derived protein, it has a problem of low stability for industrial use.

### [CITATION LIST]

### [NON PATENT LITERATURE]

[NPL 1] Pillay S., et. al., Nature, 530, 108-112, 2016
[NPL 2] Zhang. R., et. al., Nat. Microbiol., 4, 675-682, 2019

### SUMMARY

### [TECHNICAL PROBLEM]

It is an object of the invention to provide an improved AAV-binding protein having enhanced stability especially to heat, acid, and alkali, a method for producing the protein, and an AAV adsorbent using the protein.

### [SOLUTION TO PROBLEM]

In order to solve the above problems, the present inventors conducted intensive studies. As a result, the inventors identified amino acid residues involved in improving stability in AAV-binding proteins and found that variants in which the amino acid residues are substituted with different amino acid residues have excellent stability to heat, acid, and alkali. This has led to the completion of the invention.

In other words, the present application encompasses embodiments described in the following [1] to [10]:
[1] An AAV-binding protein which is selected from any one of the following (i) to (iii):
   (i) an AAV-binding protein comprising at least amino acid residues from serine at position 312 to aspartic acid at position 500 of an amino acid sequence set forth in SEQ ID NO: 1, wherein at least any one of the following amino acid substitutions (1) to (137) is present at the amino acid residues from positions 312 to 500:
      (1) a substitution of glycine with serine at position 390 of SEQ ID NO: 1,
      (2) a substitution of isoleucine with phenylalanine at position 319 of SEQ ID NO: 1,
      (3) a substitution of leucine with proline at position 321 of SEQ ID NO: 1,
      (4) a substitution of proline with leucine at position 322 of SEQ ID NO: 1,
      (5) a substitution of asparagine with aspartic acid or serine at position 324 of SEQ ID NO: 1,
      (6) a substitution of glutamine with arginine at position 327 of SEQ ID NO: 1,
      (7) a substitution of asparagine with aspartic acid or tyrosine at position 329 of SEQ ID NO: 1,
      (8) a substitution of alanine with glycine at position 330 of SEQ ID NO: 1,
      (9) a substitution of tyrosine with cysteine at position 331 of SEQ ID NO: 1,
      (10) a substitution of valine with glutamine at position 332 of SEQ ID NO: 1,
      (11) a substitution of leucine with valine or proline at position 333 of SEQ ID NO: 1,
      (12) a substitution of glutamine with arginine at position 334 of SEQ ID NO: 1,
      (13) a substitution of proline with leucine at position 337 of SEQ ID NO: 1,
      (14) a substitution of lysine with arginine or glutamic acid at position 338 of SEQ ID NO: 1,
      (15) a substitution of glutamic acid with glycine at position 340 of SEQ ID NO: 1,
      (16) a substitution of threonine with alanine at position 341 of SEQ ID NO: 1,
      (17) a substitution of tyrosine with cysteine, histidine, or asparagine at position 342 of SEQ ID NO: 1,
      (18) a substitution of threonine with methionine at position 343 of SEQ ID NO: 1,
      (19) a substitution of tyrosine with histidine at position 344 of SEQ ID NO: 1,
      (20) a substitution of aspartic acid with asparagine at position 345 of SEQ ID NO: 1,
      (21) a substitution of tryptophan with leucine or arginine at position 346 of SEQ ID NO: 1,
      (22) a substitution of glutamine with leucine or proline at position 347 of SEQ ID NO: 1,
      (23) a substitution of leucine with proline at position 348 of SEQ ID NO: 1,
      (24) a substitution of isoleucine with threonine at position 349 of SEQ ID NO: 1,
      (25) a substitution of threonine with methionine at position 350 of SEQ ID NO: 1,
      (26) a substitution of histidine with leucine at position 351 of SEQ ID NO: 1,
      (27) a substitution of proline with leucine at position 352 of SEQ ID NO: 1,
      (28) a substitution of arginine with cysteine at position 353 of SEQ ID NO: 1,
      (29) a substitution of aspartic acid with glycine at position 354 of SEQ ID NO: 1,
      (30) a substitution of tyrosine with histidine, asparagine, or cysteine at position 355 of SEQ ID NO: 1,
      (31) a substitution of serine with cysteine at position 356 of SEQ ID NO: 1,
      (32) a substitution of glycine with cysteine at position 357 of SEQ ID NO: 1,
      (33) a substitution of histidine with arginine or leucine at position 363 of SEQ ID NO: 1,
      (34) a substitution of serine with proline at position 364 of SEQ ID NO: 1,
      (35) a substitution of glutamine with arginine at position 365 of SEQ ID NO: 1,
      (36) a substitution of isoleucine with threonine at position 366 of SEQ ID NO: 1,
      (37) a substitution of leucine with proline at position 367 of SEQ ID NO: 1,
      (38) a substitution of lysine with arginine at position 368 of SEQ ID NO: 1,
      (39) a substitution of leucine with glutamine or proline at position 369 of SEQ ID NO: 1,
      (40) a substitution of serine with alanine at position 370 of SEQ ID NO: 1,
      (41) a substitution of lysine with glutamic acid at position 371 of SEQ ID NO: 1,
      (42) a substitution of threonine with alanine at position 373 of SEQ ID NO: 1,
      (43) a substitution of leucine with proline at position 376 of SEQ ID NO: 1,
      (44) a substitution of tyrosine with cysteine at position 377 of SEQ ID NO: 1,
      (45) a substitution of phenylalanine with serine at position 379 of SEQ ID NO: 1,
      (46) a substitution of valine with alanine at position 381 of SEQ ID NO: 1,
      (47) a substitution of glutamic acid with glycine at position 384 of SEQ ID NO: 1,
      (48) a substitution of asparagine with serine at position 387 of SEQ ID NO: 1,
      (49) a substitution of histidine with glutamine, leucine, or arginine at position 389 of SEQ ID NO: 1,
      (50) a substitution of glutamic acid with lysine at position 391 of SEQ ID NO: 1,
      (51) a substitution of tyrosine with cysteine at position 393 of SEQ ID NO: 1,
      (52) a substitution of valine with alanine at position 396 of SEQ ID NO: 1,
      (53) a substitution of lysine with glutamic acid or arginine at position 399 of SEQ ID NO: 1,
      (54) a substitution of arginine with serine at position 406 of SEQ ID NO: 1,
      (55) a substitution of valine with alanine at position 412 of SEQ ID NO: 1,
      (56) a substitution of glutamine with leucine at position 415 of SEQ ID NO: 1,
      (57) a substitution of phenylalanine with serine at position 416 of SEQ ID NO: 1,
      (58) a substitution of glutamine with leucine at position 441 of SEQ ID NO: 1,
      (59) a substitution of tyrosine with phenylalanine at position 442 of SEQ ID NO: 1,
      (60) a substitution of lysine with arginine at position 448 of SEQ ID NO: 1,
      (61) a substitution of glutamic acid with glycine at position 453 of SEQ ID NO: 1,
      (62) a substitution of lysine with arginine at position 455 of SEQ ID NO: 1,
      (63) a substitution of glutamic acid with glycine at position 458 of SEQ ID NO: 1,
      (64) a substitution of alanine with serine at position 461 of SEQ ID NO: 1,
      (65) a substitution of serine with arginine at position 476 of SEQ ID NO: 1,
      (66) a substitution of leucine with proline at position 477 of SEQ ID NO: 1,
      (67) a substitution of asparagine with aspartic acid at position 487 of SEQ ID NO: 1,
      (68) a substitution of asparagine with aspartic acid at position 492 of SEQ ID NO: 1,
      (69) a substitution of isoleucine with asparagine or serine at position 319 of SEQ ID NO: 1,
      (70) a substitution of threonine with isoleucine at position 320 of SEQ ID NO: 1,
      (71) a substitution of lysine with glutamic acid at position 323 of SEQ ID NO: 1,
      (72) a substitution of leucine with glutamine or proline at position 328 of SEQ ID NO: 1,
      (73) a substitution of tyrosine with histidine at position 331 of SEQ ID NO: 1,
      (74) a substitution of valine with alanine or glutamic acid at position 332 of SEQ ID NO: 1,
      (75) a substitution of proline with glutamine at position 336 of SEQ ID NO: 1,
      (76) a substitution of proline with glutamine at position 337 of SEQ ID NO: 1,
      (77) a substitution of lysine with asparagine at position 338 of SEQ ID NO: 1,
      (78) a substitution of tyrosine with arginine at position 342 of SEQ ID NO: 1,
      (79) a substitution of threonine with serine at position 350 of SEQ ID NO: 1,
      (80) a substitution of isoleucine with phenylalanine at position 366 of SEQ ID NO: 1,
      (81) a substitution of valine with alanine at position 383 of SEQ ID NO: 1,
      (82) a substitution of glutamine with arginine at position 386 of SEQ ID NO: 1,
      (83) a substitution of histidine with aspartic acid at position 389 of SEQ ID NO: 1,
      (84) a substitution of glycine with cysteine at position 392 of SEQ ID NO: 1,
      (85) a substitution of valine with alanine at position 394 of SEQ ID NO: 1,
      (86) a substitution of isoleucine with valine at position 409 of SEQ ID NO: 1,
      (87) a substitution of serine with glycine at position 476 of SEQ ID NO: 1,
      (88) a substitution of threonine with serine at position 490 of SEQ ID NO: 1,
      (89) a substitution of serine with proline at position 312 of SEQ ID NO: 1,
      (90) a substitution of alanine with serine at position 313 of SEQ ID NO: 1,
      (91) a substitution of valine with aspartic acid at position 317 of SEQ ID NO: 1,
      (92) a substitution of glutamine with proline at position 318 of SEQ ID NO: 1,
      (93) a substitution of threonine with alanine at position 320 of SEQ ID NO: 1,
      (94) a substitution of lysine with arginine at position 323 of SEQ ID NO: 1,
      (95) a substitution of valine with alanine or glutamic acid at position 326 of SEQ ID NO: 1,
      (96) a substitution of glutamine with histidine or leucine at position 327 of SEQ ID NO: 1,
      (97) a substitution of asparagine with histidine or isoleucine at position 329 of SEQ ID NO: 1,
      (98) a substitution of valine with alanine at position 332 of SEQ ID NO: 1,
      (99) a substitution of glutamic acid with glycine or valine at position 335 of SEQ ID NO: 1,
      (100) a substitution of glutamic acid with valine at position 340 of SEQ ID NO: 1,
      (101) a substitution of threonine with proline at position 341 of SEQ ID NO: 1,
      (102) a substitution of threonine with serine at position 343 of SEQ ID NO: 1,
      (103) a substitution of tyrosine with phenylalanine at position 344 of SEQ ID NO: 1,
      (104) a substitution of tryptophan with cysteine at position 346 of SEQ ID NO: 1,
      (105) a substitution of methionine with leucine at position 359 of SEQ ID NO: 1,
      (106) a substitution of glutamic acid with lysine or valine at position 360 of SEQ ID NO: 1,
      (107) a substitution of glycine with cysteine at position 361 of SEQ ID NO: 1,
      (108) a substitution of lysine with glutamic acid, asparagine, or glycine at position 362 of SEQ ID NO: 1,
      (109) a substitution of serine with leucine at position 364 of SEQ ID NO: 1,
      (110) a substitution of lysine with asparagine or aspartic acid at position 371 of SEQ ID NO: 1,
      (111) a substitution of leucine with proline or glutamine at position 372 of SEQ ID NO: 1,
      (112) a substitution of proline with leucine at position 374 of SEQ ID NO: 1,
      (113) a substitution of glutamic acid with glycine or valine at position 378 of SEQ ID NO: 1,
      (114) a substitution of phenylalanine with tyrosine or cysteine at position 379 of SEQ ID NO: 1,
      (115) a substitution of lysine with glutamic acid at position 380 of SEQ ID NO: 1,
      (116) a substitution of valine with aspartic acid at position 381 of SEQ ID NO: 1,
      (117) a substitution of isoleucine with valine at position 382 of SEQ ID NO: 1,
      (118) a substitution of glutamic acid with valine at position 384 of SEQ ID NO: 1,
      (119) a substitution of valine with aspartic acid or isoleucine at position 394 of SEQ ID NO: 1,
      (120) a substitution of asparagine with serine at position 395 of SEQ ID NO: 1,
      (121) a substitution of threonine with serine at position 397 of SEQ ID NO: 1,
      (122) a substitution of glutamic acid with valine at position 401 of SEQ ID NO: 1,
      (123) a substitution of arginine with histidine at position 403 of SEQ ID NO: 1,
      (124) a substitution of arginine with histidine at position 406 of SEQ ID NO: 1,
      (125) a substitution of glutamine with arginine at position 415 of SEQ ID NO: 1,
      (126) a substitution of threonine with alanine at position 426 of SEQ ID NO: 1,
      (127) a substitution of glutamine with leucine or arginine at position 432 of SEQ ID NO: 1,
      (128) a substitution of glutamine with arginine at position 441 of SEQ ID NO: 1,
      (129) a substitution of histidine with leucine at position 443 of SEQ ID NO: 1,
      (130) a substitution of lysine with glutamic acid at position 448 of SEQ ID NO: 1,
      (131) a substitution of isoleucine with valine at position 456 of SEQ ID NO: 1,
      (132) a substitution of aspartic acid with asparagine at position 483 of SEQ ID NO: 1,
      (133) a substitution of serine with leucine at position 488 of SEQ ID NO: 1,
      (134) a substitution of valine with glutamic acid or isoleucine at position 499 of SEQ ID NO: 1;
   (ii) an AAV-binding protein comprising at least amino acid residues from serine at position 312 to aspartic acid at position 500 of the amino acid sequence set forth in SEQ ID NO: 1, wherein at least any one of the amino acid substitutions (1) to (134) is present at the amino acid residues from positions 312 to 500, wherein one or more of substitutions, deletions, insertions, and additions of one or several amino acid residues are further present at one or several positions other than the amino acid substitutions shown in (1) to (134), and wherein the AAV-binding protein has AAV-binding activity; and
   (iii) an AAV-binding protein comprising an amino acid sequence, wherein the amino acid sequence has 70% or more homology to an entire amino acid sequence in which at least any one of the amino acid substitutions (1) to (134) is present in an amino acid sequence ranging from serine at position 312 to aspartic acid at position 500 of the amino acid sequence set forth in SEQ ID NO: 1, wherein the at least any one of the amino acid substitutions remains in the amino acid sequence, and wherein the AAV-binding protein has AAV-binding activity.
[2] The AAV-binding protein according to [1], which is selected from any one of the following (iv) to (vi):
   (iv) an AAV-binding protein comprising at least amino acid residues from serine at position 312 to aspartic acid at position 500 of the amino acid sequence set forth in SEQ ID NO: 1, wherein at least the following amino acid substitution (1) is present at the amino acid residues from positions 312 to 500:
      (1) a substitution of glycine with serine at position 390 of SEQ ID NO: 1;
   (v) an AAV-binding protein comprising at least amino acid residues from serine at position 312 to aspartic acid at position 500 of the amino acid sequence set forth in SEQ ID NO: 1, wherein at least the amino acid substitution (1) is present at the amino acid residues from positions 312 to 500, wherein one or more of substitutions, deletions, insertions, and additions of one or several amino acid residues are further present at one or several positions other than the amino acid substitution shown in (1), and wherein the AAV-binding protein has AAV-binding activity; and
   (vi) an AAV-binding protein comprising an amino acid sequence, wherein the amino acid sequence has 70% or more homology to an entire amino acid sequence in which at least the amino acid substitution (1) is present in an amino acid sequence ranging from serine at position 312 to aspartic acid at position 500 of the amino acid sequence set forth in SEQ ID NO: 1, wherein the amino acid substitution (1) remains in the amino acid sequence, and wherein the AAV-binding protein has AAV-binding activity.
[3] The AAV-binding protein according to [2], wherein at least any one of amino acid substitutions shown in the following (A) to (K) is present:
   (A) a substitution of glycine with serine at position 390 of SEQ ID NO: 1 and a substitution of tyrosine with cysteine at position 342 of SEQ ID NO: 1,
   (B) a substitution of glycine with serine at position 390 of SEQ ID NO: 1, a substitution of tyrosine with cysteine at position 342 of SEQ ID NO: 1, and a substitution of serine with arginine at position 476 of SEQ ID NO: 1,
   (C) a substitution of glycine with serine at position 390 of SEQ ID NO: 1, a substitution of isoleucine with phenylalanine at position 319 of SEQ ID NO: 1, a substitution of tyrosine with cysteine at position 342 of SEQ ID NO: 1, and a substitution of serine with arginine at position 476 of SEQ ID NO: 1,
   (D) a substitution of glycine with serine at position 390 of SEQ ID NO: 1, a substitution of tyrosine with cysteine at position 342 of SEQ ID NO: 1, a substitution of serine with arginine at position 476 of SEQ ID NO: 1, and a substitution of asparagine with aspartic acid at position 487 of SEQ ID NO: 1,
   (E) a substitution of glycine with serine at position 390 of SEQ ID NO: 1, a substitution of isoleucine with phenylalanine at position 319 of SEQ ID NO: 1, a substitution of lysine with glutamic acid at position 323 of SEQ ID NO: 1, a substitution of tyrosine with cysteine at position 342 of SEQ ID NO: 1, and a substitution of serine with arginine at position 476 of SEQ ID NO: 1,
   (F) a substitution of glycine with serine at position 390 of SEQ ID NO: 1, a substitution of tyrosine with cysteine at position 342 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
   (G) a substitution of glycine with serine at position 390 of SEQ ID NO: 1, a substitution of asparagine with aspartic acid at position 324 of SEQ ID NO: 1, a substitution of tyrosine with cysteine at position 342 of SEQ ID NO: 1, a substitution of lysine with glutamic acid at position 362 of SEQ ID NO: 1,a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
   (H) a substitution of glycine with serine at position 390 of SEQ ID NO: 1, a substitution of valine with aspartic acid at position 317 of SEQ ID NO: 1, a substitution of tyrosine with cysteine at position 342 of SEQ ID NO: 1, a substitution of lysine with asparagine at position 371 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
   (I) a substitution of glycine with serine at position 390 of SEQ ID NO: 1, a substitution of valine with aspartic acid at position 317 of SEQ ID NO: 1, a substitution of asparagine with aspartic acid at position 324 of SEQ ID NO: 1, a substitution of tyrosine with cysteine at position 342 of SEQ ID NO: 1, a substitution of lysine with glutamic acid at position 362 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
   (J) a substitution of glycine with serine at position 390 of SEQ ID NO: 1, a substitution of valine with aspartic acid at position 317 of SEQ ID NO: 1, a substitution of tyrosine with cysteine at position 342 of SEQ ID NO: 1, a substitution of lysine with glutamic acid at position 362 of SEQ ID NO: 1, a substitution of lysine with asparagine at position 371 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1, and
   (K) a substitution of glycine with serine at position 390 of SEQ ID NO: 1, a substitution of valine with aspartic acid at position 317 of SEQ ID NO: 1, a substitution of tyrosine with serine at position 342 of SEQ ID NO: 1, a substitution of lysine with glutamic acid at position 362 of SEQ ID NO: 1, a substitution of lysine with asparagine at position 371 of SEQ ID NO: 1, a substitution of lysine with alanine at position 381 of SEQ ID NO: 1, a substitution of isoleucine with valine at position 382 of SEQ ID NO: 1, a substitution of glycine with serine at position 390 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1.
[4] The AAV-binding protein according to [3], which is selected from any one of the following (vii) to (ix):
   (vii) an AAV-binding protein comprising at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76;
   (viii) an AAV-binding protein comprising at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76, wherein one or more of substitutions, deletions, insertions, and additions of one or several amino acid residues are further present at one or several positions of the amino acid residues from positions 25 to 213 other than the amino acid substitution in the amino acid sequence, and wherein the AAV-binding protein has AAV-binding activity; and
   (ix) an AAV-binding protein comprising amino acid residues having 70% or more homology to at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76, wherein one or more amino acid substitutions of said amino acid sequence are present, and having AAV-binding activity.
[5] A polynucleotide encoding the AAV-binding protein according to any one of [1] to [4].
[6] An expression vector comprising the polynucleotide according to [5].
[7] A transformant obtained by transforming a host with the expression vector according to [6].
[8] The transformant according to [7], wherein the host is *Escherichia coli.*
[9] A method for producing an AAV-binding protein, comprising a step of allowing the AAV-binding protein to be expressed by culturing the transformant according to [7] or [8] and a step of recovering the AAV-binding protein expressed from the obtained culture.
[10] An AAV adsorbent comprising an insoluble carrier and the AAV-binding protein according to any one of [1] to [3] which is immobilized on the carrier.
[11] A column containing the AAV adsorbent according to [10].
[12] A method for purifying AAV, comprising a step of adding a solution containing AAV to the column according to [11] to allow the AAV to be adsorbed by the adsorbent and a step of eluting the AAV adsorbed by the adsorbent using an eluate.

Hereinafter, the invention will be described in detail. However, the invention is not bound by the following embodiments, and can be implemented in any embodiment as long as the gist of the invention is not deviated.

The patent publications, patent applications, unexamined patent publications, non-patent literature, and the like cited herein are all incorporated into the invention for all purposes by reference in their entirety.

The AAV binding protein described herein is a protein comprising amino acid residues from serine (Ser) at position 312 to aspartic acid (Asp) at position 500, which are the regions corresponding to the extracellular domains 1 (PKD1) and 2 (PKD2), in the amino acid sequence of KIAA0319L (official database: UniProt; Accession Number: Q8IZA0) set forth in SEQ ID NO: 1, in which an amino acid substitution is present at a specific position of the amino acid residues from positions 312 to 500. Therefore, the AAV-binding protein of the invention may comprise: all or part of the other extracellular domains (domain 3 (PKD3), domain 4 (PKD4), and domain 5 (PKD5)) on the C-terminal side of the protein; all or part of a signal sequence such as the MANSC (motif at N terminus with seven cysteines) domain on the N-terminal side of PKD1 or cysteine-rich region; and all or part of the transmembrane and intracellular domains on the N-terminal and/or C-terminal side of the extracellular domain.

It is preferable that there is the amino acid substitution at a specific position, which is specifically at least one amino acid substitution of Ile319Phe (this notation indicates a substitution of isoleucine at position 319 of SEQ ID NO: 1 with phenylalanine; the same applies hereinafter), Leu321Pro, Pro322Leu, Asn324Asp, Asn324Ser, Gln327Arg, Asn329Asp, Asn329Tyr, Ala330Gly, Tyr331Cys, Val332Gln, Leu333Val, Leu333Pro, Gln334Arg, Pro337Leu, Lys338Arg, Lys338Glu, Glu340Gly, Thr341Ala, Tyr342Cys, Tyr342His, Tyr342Asn, Th
r343Met, Tyr344His, Asp345Asn, Trp346Leu, Trp346Arg, Gln347Leu, Gln347Pro, Leu348Pro, Ile349Thr, Thr350Met, His351Leu, Pro352Leu, Arg353Cys, Asp354Gly, Tyr355His, Tyr355Asn, Tyr355Cys, Ser356Cys, Gly357Cys, His363Arg, His363Leu, Ser364Pro, Gln365Arg, Ile366Thr, Leu367Pro, Lys368Arg, Leu369Gln, Leu369Pro, Ser370Ala, Lys371Glu, Thr373Ala, Leu376Pro, Tyr377Cys, Phe379Ser, Val381Ala, Glu384Gly, Asn387Ser, His389Gln, His389Leu, His389Arg, Gly390Ser, Glu391Lys, Tyr393Cys, Val396Ala, Lys399Glu, Lys399Arg, Arg406Ser, Val412Ala, Gln415Leu, Phe416Ser, Gln441Leu, Tyr442Phe, Lys448Arg, Glu453Gly, Lys455Arg, Glu458Gly, Ala461Ser, Ser476Arg, Leu477Pro, Asn487Asp, and Asn492Asp in the amino acid sequence set forth in SEQ ID NO: 1 such that the stability to heat and/or acid is improved.

Of these, Gly390Ser is an amino acid substitution with particularly improved stability to heat and acid. Therefore, an AAV-binding protein in which at least the amino acid substitution of Gly390Ser is present is a preferred embodiment of the AAV-binding protein of the invention.

It is also preferable that there is at least one amino acid substitution of Ile319Asn, Ile319Ser, Thr320Ile, Lys323Glu, Leu328Gln, Leu328Pro, Tyr331His, Val332Ala, Val332Glu, Pro336Gln, Pro337Gln, Lys338Asn, Tyr342Arg, Thr350Ser, Ile366Phe, Val383Ala, Gln386Arg, His389Asp, Gly392Cys, Val394Ala, Ile409Val, Ser476Gly, and Thr490Ser in the amino acid sequence set forth in SEQ ID NO: 1 such that the stability to heat and/or acid is improved.

It is still also preferable that there is at least one amino acid substitution of Val317Asp, Gln318Pro, Thr320Ala, Val326Ala, Val326Glu, Leu328Pro, Asn329His, Val332Ala, Lys362Glu, Lys362Asn, Lys371Asn, Leu376Pro, Glu378Gly, Glu378Val, Phe379Tyr, Phe379Ser, Val381Asp, Thr397Ser, Glu401Val, Arg406His, and Val499Glu in the amino acid sequence set forth in SEQ ID NO: 1 such that the stability to heat and/or acid is improved.

It is further preferable that there is at least one amino acid substitution of Ser312Pro, Ala313Ser, Lys323Arg, Asn324Ser, Gln327His, Gln327Leu, Asn329Ile, Glu335Gly, Glu335Val, Glu340Val, Thr341Pro, Thr343Ser, Tyr344Phe, Trp346Cys, Arg353Cys, Tyr355Cys, Ser356Cys, Gly357Cys, Met359Leu, Glu360Lys, Glu360Val, Gly361Cys, Lys362Gly, Ser364Leu, Ile366Thr, Leu369Gln, Lys371Asp, Leu372Pro, Leu372Gln, Thr373Ala, Pro374Leu, Leu376Pro, Glu378Gly, Glu378Val, Phe379Cys, Phe379Ser, Lys380Glu, Val381Ala, Ile382Val, Val383Ala, Glu384Val, Gln386Arg, His389Arg, Tyr393Cys, Val394Asp, Val394Ile, Asn395Ser, Val396Ala, Arg403His, Gln415Arg, Thr426Ala, Gln432Leu, Gln432Arg, Gln441Arg, His443Leu, Lys448Glu, Ile456Val, Asp483Asn, Ser488Leu, and Val499Ile in the amino acid sequence set forth in SEQ ID NO: 1 such that the stability to heat and/or alkali is improved.

In the AAV-binding protein of the invention, the number of amino acids to be substituted is not particularly limited. As an example, the AAV-binding proteins described in the following (A) to (K) can be mentioned. These AAV-binding proteins are more preferable in that they have improved heat and acid stability.
(A) An AAV-binding protein comprising amino acid residues from serine at position 312 to aspartic acid at position 500 of the amino acid sequence set forth in SEQ ID NO: 1, wherein at least amino acid substitutions of Gly390Ser and Tyr342Cys are present at the amino acid residues from positions 312 to 500.
(B) An AAV-binding protein comprising amino acid residues from serine at position 312 to aspartic acid at position 500 of the amino acid sequence set forth in SEQ ID NO: 1, wherein at least amino acid substitutions of Gly390Ser, Tyr342Cys, and Ser476Arg are present at the amino acid residues from positions 312 to 500.
(C) An AAV-binding protein comprising amino acid residues from serine at position 312 to aspartic acid at position 500 of the amino acid sequence set forth in SEQ ID NO: 1, wherein at least amino acid substitutions of Gly390Ser, Ile319Phe, Tyr342Cys, and Ser476Arg are present at the amino acid residues from positions 312 to 500.
(D) An AAV-binding protein comprising amino acid residues from serine at position 312 to aspartic acid at position 500 of the amino acid sequence set forth in SEQ ID NO: 1, wherein at least amino acid substitutions of Gly390Ser, Tyr342Cys, Ser476Arg, and Asn487Asp are present at the amino acid residues from positions 312 to 500.
(E) An AAV-binding protein comprising amino acid residues from serine at position 312 to aspartic acid at position 500 of the amino acid sequence set forth in SEQ ID NO: 1, wherein at least amino acid substitutions of Gly390Ser, Ile319Phe, Lys323Glu, Tyr342Cys, and Ser476Arg are present at the amino acid residues from positions 312 to 500.
(F) An AAV-binding protein comprising amino acid residues from serine at position 312 to aspartic acid at position 500 of the amino acid sequence set forth in SEQ ID NO: 1, wherein at least amino acid substitutions of Gly390Ser, Tyr342Cys, Lys399Glu, Ser476Arg, and Asn487Asp are present at the amino acid residues from positions 312 to 500.
(G) An AAV-binding protein comprising amino acid residues from serine at position 312 to aspartic acid at position 500 of the amino acid sequence set forth in SEQ ID NO: 1, wherein at least amino acid substitutions of Gly390Ser, Asn324Asp, Tyr342Cys, Lys362Glu, Lys399Glu, Ser476Arg, and Asn487Asp are present at the amino acid residues from positions 312 to 500.
(H) An AAV-binding protein comprising amino acid residues from serine at position 312 to aspartic acid at position 500 of the amino acid sequence set forth in SEQ ID NO: 1, wherein at least amino acid substitutions of Gly390Ser, Val317Asp, Tyr342Cys, Lys371Asn, Lys399Glu, Ser476Arg, and Asn487Asp are present at the amino acid residues from positions 312 to 500.
(I) An AAV-binding protein comprising amino acid residues from serine at position 312 to aspartic acid at position 500 of the amino acid sequence set forth in SEQ ID NO: 1, wherein at least amino acid substitutions of Gly390Ser, Val317Asp, Asn324Asp, Tyr342Cys, Lys362Glu, Lys399Glu, Ser476Arg, and Asn487Asp are present at the amino acid residues from positions 312 to 500.
(J) An AAV-binding protein comprising amino acid residues from serine at position 312 to aspartic acid at position 500 of the amino acid sequence set forth in SEQ ID NO: 1, wherein at least amino acid substitutions of Gly390Ser, Val317Asp, Tyr342Cys, Lys362Glu, Lys371Asn, Lys399Glu, Ser476Arg, and Asn487Asp are present at the amino acid residues from positions 312 to 500.
(K) An AAV-binding protein comprising amino acid residues from serine at position 312 to aspartic acid at position 500 of the amino acid sequence set forth in SEQ ID NO: 1, wherein at least amino acid substitutions of Gly390Ser, Val317Asp, Tyr(Cys)342Ser, Lys362Glu, Lys371Asn, Val381Ala, Ile382Val, Lys399Glu, Ser476Arg, and Asn487Asp are present at the amino acid residues from positions 312 to 500.

More specifically, examples of the AAV-binding protein of the invention include the following AAV-binding proteins. These AAV-binding proteins are preferable in that they have improved heat and acid stability.
An AAV-binding protein comprising at least an amino acid sequence ranging from serine at position 25 to aspartic acid at position 213 of the amino acid sequence set forth in SEQ ID NO: 18, which is one aspect of the AAV-binding protein shown in (A).
An AAV-binding protein comprising at least an amino acid sequence ranging from serine at position 25 to aspartic acid at position 213 of the amino acid sequence set forth in SEQ ID NO: 32, which is one aspect of the AAV-binding protein shown in (B).
An AAV-binding protein comprising at least an amino acid sequence ranging from serine at position 25 to aspartic acid at position 213 of the amino acid sequence set forth in SEQ ID NO: 34, which is one aspect of the AAV-binding protein shown in (C).
An AAV-binding protein comprising at least an amino acid sequence ranging from serine at position 25 to aspartic acid at position 213 of the amino acid sequence set forth in SEQ ID NO: 38, which is one aspect of the AAV-binding protein shown in (D).
An AAV-binding protein comprising at least an amino acid sequence ranging from serine at position 25 to aspartic acid at position 213 of the amino acid sequence set forth in SEQ ID NO: 42, which is one aspect of the AAV-binding protein shown in (E).
An AAV-binding protein comprising at least an amino acid sequence ranging from serine at position 25 to aspartic acid at position 213 of the amino acid sequence set forth in SEQ ID NO: 46, which is one aspect of the AAV-binding protein shown in (F).
An AAV-binding protein comprising at least an amino acid sequence ranging from serine at position 25 to aspartic acid at position 213 of the amino acid sequence set forth in SEQ ID NO: 53, which is one aspect of the AAV-binding protein shown in (G).
An AAV-binding protein comprising at least an amino acid sequence ranging from serine at position 25 to aspartic acid at position 213 of the amino acid sequence set forth in SEQ ID NO: 55, which is one aspect of the AAV-binding protein shown in (H).
An AAV-binding protein comprising at least an amino acid sequence ranging from serine at position 25 to aspartic acid at position 213 of the amino acid sequence set forth in SEQ ID NO: 59, which is one aspect of the AAV-binding protein shown in (I).
An AAV-binding protein comprising at least an amino acid sequence ranging from serine at position 25 to aspartic acid at position 213 of the amino acid sequence set forth in SEQ ID NO: 63, which is one aspect of the AAV-binding protein shown in (J).
An AAV-binding protein comprising at least an amino acid sequence ranging from serine at position 25 to aspartic acid at position 231 of the amino acid sequence set forth in SEQ ID NO: 69, which is one aspect of the AAV-binding protein shown in (K).

In addition, more specifically, examples of the AAV-binding protein of the invention include the following AAV-binding proteins. The AAV-binding proteins described are preferable in that they have improved heat stability.
An AAV-binding protein comprising at least an amino acid sequence ranging from serine at position 25 to aspartic acid at position 213 of the amino acid sequence set forth in SEQ ID NO: 76, which is one aspect of the AAV-binding protein shown in (F).

In (ii), (v), and (viii) above, the expression "one or several" refers to, for example, from 1 to 50, from 1 to 30, from 1 to 20, from 1 to 10, from 1 to 9, from 1 to 8, from 1 to 7, from 1 to 6, from 1 to 5, from 1 to 4, from 1 to 3, from 1 to 2, or 1, although it depends on positions of amino acid substitutions and types of amino acid residues in the protein conformation of the AAV-binding protein. One example of an amino acid substitution is a conservative substitution between amino acids having similar physical and/or chemical properties. Those skilled in the art know that in the case of conservative substitution, the protein function is usually maintained between a protein having a substitution and a protein lacking the substitution. One example of a conservative substitution is a substitution that occurs between glycine and alanine, serine and proline, or glutamic acid and alanine (Protein Structure and Function, Medical Sciences International, Ltd., 9, 2005). Another example of the amino acid substitution is a substitution for monomerizing the AAV-binding protein of the invention. Specifically, examples thereof include amino acid substitutions in which a cysteine residue that easily constitutes a higher-order structure is substituted with a serine residue or a methionine residue.

Further, any one or more of substitutions, deletions, insertions, and additions described herein also include naturally occurring mutations (mutants or variants) based on the difference in the origin of AAV-binding proteins the difference in species, and the like.

The homology of the amino acid sequences in (iii), (vi), and (ix) above may be 70% or more, and the homology may be more than that (e.g., 80% or more, 85% or more, 90% or more, 95%, or 98). The term "homology" used herein may refer to similarity or identity and may particularly refer to identity. The "amino acid sequence homology" means homology to the entire amino acid sequence. The "identity" between amino acid sequences means the proportion of amino acid residues of the same type in those amino acid sequences (Experimental Medicine, February 2013, Vol. 31, No. 3, YODOSHA CO., LTD.). The "similarity" between amino acid sequences means the sum of the proportion of amino acid residues of the same type in those amino acid sequences and the proportion of amino acid residues having similar side chain properties (Experimental Medicine, February 2013, Vol. 31, No. 3, YODOSHA CO., LTD.). The homology of the amino acid sequence can be determined by using an alignment program such as BLAST (Basic Local Alignment Search Tool) or FASTA.

It is also possible to further add a useful oligopeptide to the N-terminal side or the C-terminal side of the AAV-binding protein of the invention for separation from a solution with the presence of contaminants. Examples of the oligopeptide include polyhistidine, polylysine, polyarginine, polyglutamic acid, and polyaspartic acid. In addition, a cysteine-containing oligopeptide useful for immobilizing the AAV-binding protein of the invention to a solid phase such as a support for chromatography (e.g., an oligopeptide consisting of amino acid residues from cysteine at position 220 to glycine at position 226 of the amino acid sequence set forth in SEQ ID NO: 50.) may be further added to the N-terminal side or the C-terminal side of the AAV-binding protein of the invention.

The length of an oligopeptide to be added to the N-terminal side or the C-terminal side of the AAV-binding protein is not particularly limited as long as the AAV-binding property and stability of the AAV-binding protein of the invention are not impaired. When adding the oligopeptide to the AAV-binding protein of the invention, it is possible to prepare a polynucleotide encoding the oligopeptide, and then, add the polynucleotide to the N-terminal side or the C-terminal side of the AAV-binding protein by a genetic engineering method known to those skilled in the art. Alternatively, it is also possible to chemically synthesize the oligopeptide and add the oligopeptide to the N-terminal side or the C-terminal side of the AAV-binding protein of the invention via chemical binding.

It is also possible to further add a signal peptide for promoting efficient expression in a host to the N-terminal side of the AAV-binding protein of the invention. Examples of the signal peptide when the host is *Escherichia coli* (*E. coli)*can include signal peptides that protein secretion in a periplasmic space, such as PelB, OmpA, DsbA, DsbC, MalE, and TorT (Japanese Unexamined Patent Publication (Kokai) No. 2011-097898).

The expression such as "with enhanced stability especially to heat, acid, or alkali" used herein may mean improved stability to heat, acid, or alkali compared to a wild-type AAV-binding protein (i.e., a protein comprising at least amino acid residues from serine at position 25 to aspartic acid at position 213 in the amino acid sequence set forth in SEQ ID NO: 7), preferably improved stability to heat and acid or alkali, more preferably improved stability to heat, acid, and alkali.

Examples of a method for producing a polynucleotide encoding the AAV-binding protein of the invention (hereinafter referred to as "polynucleotide of the invention") can include:
(I) a method for artificially synthesizing a polynucleotide comprising a nucleotide sequence that is converted from the amino acid sequence of the AAV-binding protein of the invention; and
(II) a method in which a polynucleotide comprising the entire or partial sequence of the AAV-binding protein is prepared directly in an artificial manner or by a DNA amplification method such as a PCR method using cDNA or the like of the AAV-binding protein, and the prepared polynucleotide is ligated in a suitable manner.

In the method (I) described above, when converting from an amino acid sequence to a nucleotide sequence, it is preferable to perform the conversion in consideration of the frequency of use of codons in the host to be transformed. In one example, when the host is *E. coli,* the conversion may be performed to avoid codons such as AGA/AGG/CGG/CGA for arginine (Arg), ATA for isoleucine (Ile), CTA for leucine (Leu), GGA for glycine (Gly), and CCC for proline (Pro) because those codons are used infrequently (they are so-called rare codons). Analysis of the frequency of codon usage is also possible by using a public database (e.g., the Codon Usage Database on the website of Kazusa DNA Research Institute).

When introducing a mutation into the polynucleotide of the invention, the error-prone PCR method can be used. The reaction conditions in the error-prone PCR method are not particularly limited as long as the desired mutation can be introduced into the polynucleotide encoding the AAV-binding protein. For example, the mutation can be introduced by, for example, making the concentration of four kinds of deoxynucleotides (dATP/dTTP/dCTP/dGTP) serving as substrates different, adding MnCl₂ at a concentration of from 0.01 to 10 mM (preferably from 0.1 to 1 mM) to a PCR reaction solution, and performing PCR. In addition, examples of a mutagenesis method other than the error prone PCR method include a method for producing a mutant by allowing an agent serving as a mutagen to come into contact with or act on a polynucleotide comprising the entire or partial sequence of an AAV-binding protein or irradiating the polynucleotide with ultraviolet rays to introduce a mutation into the polynucleotide. As an agent used as a mutagen in the method, a mutagenic agent commonly used by those skilled in the art such as hydroxylamine, N-methyl-N'-nitro-N-nitrosoguanidine, nitrite, sulfurous acid, or hydrazine may be used.

When transforming a host with the polynucleotide of the invention, the polynucleotide of the present invention itself may be used. However, it is more preferable to use a polynucleotide obtained by inserting the polynucleotide of the invention at an appropriate position in an expression vector (e.g., bacteriophage, cosmid, plasmid, or the like commonly used for transformation of prokaryotic or eukaryotic cells). The expression vector is not particularly limited as long as it exists stably and can be replicated in the host to be transformed. When *E*. *coli* is used as the host, examples thereof can include a pET plasmid vector, a pUC plasmid vector, a pTrc plasmid vector, and a pCDF plasmid vector.

In addition, the appropriate position means a position that does not destroy the replication function of the expression vector, the desired antibiotic marker, or the transmissible region. When inserting the polynucleotide of the invention into the expression vector, it is preferable to insert it in a state of being linked to a functional polynucleotide such as a promoter required for expression. When the host is *E. coli,* examples of the promoter include a trp promoter, a tac promoter, a trc promoter, a lac promoter, a T7 promoter, a recA promoter, and an lpp promoter.

The host may be transformed using the expression vector having the polynucleotide of the invention inserted (hereinafter referred to as "expression vector of the invention") by a method usually used by those skilled in the art. For example, when a microorganism belonging to the genus *Escherichia* (e.g., *E. coli* JM109 strain, *E*. *coli* BL21 (DE3) strain, or *E*. *coli* W3110 strain) is used as the host, it may be transformed by a method described in a known document (e.g., Molecular Cloning, Cold Spring Harbor Laboratory,256,1992) or the like. Transformants obtained via transformation by the method described above are screened by an appropriate method such that a transformant capable of expressing the AAV-binding protein of the invention (hereinafter referred to as "transformant of the invention") can be obtained.

The expression vector of the invention from the transformant of the invention may be prepared from the culture obtained by culturing the transformant of the invention by an alkaline extraction method or a commercially available extraction kit such as a QIAprep Spin Miniprep kit (manufactured by QIAGEN).

The AAV-binding protein of the invention can be produced by culturing the transformant of the invention and recovering the AAV-binding protein of the invention from the obtained culture. The culture described herein includes not only the cultured cells of the transformant of the invention itself, but also the medium used for the culture.

The transformant used in the method for producing the protein of the invention may be cultured in a medium suitable for culturing a target host. When the host is *E. coli,* LB (Luria-Bertani) medium supplemented with necessary nutrient sources is given as an example of a preferable medium. To selectively grow the transformant of the invention depending on whether or not the vector of the invention is introduced, it is preferable to add a drug corresponding to a drug resistance gene contained in the vector to the medium and culture the transformant. For example, when the vector contains a kanamycin resistance gene, kanamycin may be added to the medium.

In addition to carbon, nitrogen, and inorganic salt sources, suitable nutrient sources may be added to the medium. The medium may contain one or more reducing agents optionally selected from the group consisting of glutathione, cysteine, cystamine, thioglycolate, and dithiothreitol. When the host is *E. coli,* the culture temperature is generally from 10°C to 40°C, preferably from 20°C to 37°C, more preferably around 25°C, but may be selected depending on the characteristics of the protein to be expressed. When the host is *E. coli,* the pH of the medium is pH 6.8 to pH 7.4, preferably around pH 7.0. When the vector of the invention contains an inducible promoter, it is preferable to induce the vector under conditions that allow favorable expression of the AAV-binding protein of the invention.

As the inducer, isopropyl-β-D-thiogalactopyranoside (IPTG) can be exemplified. When the host is *E. coli,* the turbidity of the culture solution (absorbance at 600 nm) is measured, and when it becomes about 0.5 to 1.0, an appropriate amount of IPTG is added and then the culture is continued, thereby achieving the expression of the AAV-binding protein. The concentration of IPTG added may be appropriately selected from a range of from 0.005 to 1.0 mM, preferably a range of from 0.01 to 0.5 mM. Various conditions relating to IPTG induction may be performed under conditions well known in the art.

To recover the AAV-binding protein of the invention from the culture obtained by culturing the transformant of the invention, the AAV-binding protein of the invention may be recovered by separation/purification from the culture by a method suitable for the expression form of the AAV-binding protein of the invention in the transformant of the invention. For example, when the AAV-binding protein of the invention is expressed in the culture supernatant, the cells may be separated by centrifugation and the AAV-binding protein may be purified from the obtained culture supernatant. In addition, when the AAV-binding protein of the invention is expressed intracellularly (in a periplasmic space), after collecting the cells by centrifugation, the cells are disrupted by adding an enzyme treatment agent, a surfactant, or the like to extract the AAV-binding protein, and then the AAV-binding protein may be purified.

To purify the AAV-binding protein of the invention, a method known in the art may be used, and one example thereof is separation/purification using liquid chromatography. Examples of liquid chromatography include on ion exchange chromatography, hydrophobic interaction chromatography, gel filtration chromatography, and affinity chromatography. By performing a purification operation in combination with these chromatography methods, the AAV-binding protein of the invention can be prepared with high purity.

As a method for measuring the binding activity of the obtained AAV-binding protein of the invention to AAV, for example, the binding activity to AAV may be measured by using an enzyme-linked immunosorbent assay method (hereinafter referred to as "ELISA method"), a surface plasmon resonance method, or the like. The AAV used for measuring the binding activity may be an AAV vector or a VLP (virus-like particle). In addition, any serotype of AAV vector and of VLP may be used as long as it exhibits binding activity to the AAV-binding protein of the invention.

The AAV-binding protein of the invention can be used, for example, for purification or analysis of AAV. The AAV-binding protein of the invention can be used, for example, by immobilizing it on an insoluble carrier. In other words, purification or analysis of AAV can be specifically performed using, an AAV adsorbent comprising, for example, an insoluble carrier and the AAV-binding protein of the invention immobilized on the insoluble carrier. The AAV adsorbent of the present invention described herein also refers to an AAV adsorbent comprising the insoluble carrier and the AAV-binding protein of the invention immobilized on the insoluble carrier. The purification of AAV is not limited to the purification of AAV from a solution in which contaminants coexist, but also includes the purification of AAV based on its structure, properties, activity, and the like. An insoluble carrier is not particularly limited. Examples thereof include carriers made with polysaccharides such as agarose, alginate (alginic acid salt), carrageenan, chitin, cellulose, dextrin, dextran, and starch, carriers made with synthetic polymers such as polyvinyl alcohol, polymethacrylate, poly(2-hydroxyethyl methacrylate), and polyurethane, and carriers made with ceramics such as silica. Of these, carriers made with polysaccharides and carriers made with synthetic polymers are preferable as insoluble carriers. Examples of the preferable carrier include hydroxylated polymethacrylate gel such as TOYOPEARL (manufactured by Tosoh Corporation), agarose gel such as Sepharose (manufactured by Cytiva), and cellulose gel such as Cellufine (manufactured by JNC). The shape of insoluble carrier is not particularly limited. The insoluble carrier may have, for example, a shape that can be filled in a column. The insoluble carrier may be, for example, a granular substance, a monolith-like substance, a film-like substance, a fibrous substance, or the like. Further, the insoluble carrier may be, for example, porous or non-porous.

The AAV-binding protein of the invention can be immobilized on an insoluble carrier, for example, via a covalent bond. Specifically, the AAV-binding protein of the invention can be immobilized on an insoluble carrier by covalently binding the AAV-binding protein of the invention and the insoluble carrier, for example, via an active group of the insoluble carrier. In other words, the insoluble carrier may have an active group. The insoluble carrier may have, for example, an active group on its surface. Examples of an active group include an N-hydroxy succinimide (NHS)-activated ester group, an epoxy group, a carboxy group, a maleimide group, a haloacetyl group, a tresyl group, a formyl group, and a haloacetamide group. As the insoluble carrier having an active group, for example, a commercially available insoluble carrier having an active group may be used as it is, or an active group may be introduced into the insoluble carrier for use. Examples of a commercially available carrier having an active group may include TOYOPEARL AF-Epoxy-650M, TOYOPEARL AF-Tresyl-650M (each manufactured by Tosoh Corporation), HiTrap NHS-activated HP Columns, NHS-activated Sepharose 4 Fast Flow, Epoxy-activated Sepharose 6B (each manufactured by Cytiva), and SulfoLink Coupling Resin (manufactured by Thermo Fisher Scientific).

As a method for introducing an active group on the carrier surface, a method in which a compound having two or more active sites is allowed to react, at one of the active sites, with a hydroxy group, an epoxy group, a carboxy group, an amino group, or the like present on the carrier surface can be exemplified.

Examples of a compound for introducing an epoxy group to a hydroxy group or an amino group present on the carrier surface may include epichlorohydrin, ethanediol diglycidyl ether, butanediol diglycidyl ether, and hexanediol diglycidyl ether.

Examples of a compound for introducing a carboxy group to an epoxy group present on the carrier surface may include 2-mercaptoacetic acid, 3-mercaptopropionic acid, 4-mercaptobutyric acid, 6-mercaptobutyric acid, glycine, 3-aminopropionic acid, 4-aminobutyric acid, and 6-aminohexanoic acid.

Examples of a compound for introducing a maleimide group to a hydroxy group, an epoxy group, a carboxy group, or an amino group present on the carrier surface may include N-(ε-maleimidocaproic acid)hydrazide, N-(ε-maleimidopropionic acid)hydrazide, 4-(4-N-maleimidophenyl)acetic acid hydrazide, 2-aminomaleimide, 3-aminomaleimide, 4-aminomaleimide, 6-aminomaleimide, 1-(4-aminophenyl)maleimide, 1-(3-aminophenyl)maleimide, 4-(maleimide)phenyl isocyanate, 2-maleimidoacetic acid, 3-maleimidopropionic acid, 4-maleimidobutyric acid, 6-maleimidohexanoic acid, N-(α-maleimidoacetoxy)succinimide ester, (m-maleimidobenzoyl)N-hydroxy succinimide ester, succinimidyl-4-(maleimidomethyl)cyclohexane-1-carbonyl-(6-aminohexanoic acid), succinimidyl-4-(maleimidomethyl)cyclohexane-1-carboxylic acid, (p-maleimidobenzoyl)N-hydroxy succinimide ester, and (m-maleimidobenzoyl)N-hydroxy succinimide ester.

Examples of a compound for introducing a haloacetyl group to a hydroxy group or an amino group present on the carrier surface may include chloroacetic acid, bromoacetic acid, iodoacetic acid, chloroacetic acid chloride, bromoacetic acid chloride, bromoacetic acid bromide, chloroacetic acid anhydride, bromoacetic acid anhydride, iodoacetic acid anhydride, 2-(iodoacetamido)acetic acid-N-hydroxysuccinimide ester, 3-(bromoacetamido)propionic acid-N-hydroxysuccinimide ester, and 4-(iodoacetyl)aminobenzoic acid-N-hydroxysuccinimide ester.

As a method for introducing an active group on the carrier surface, a method in which ω-alkenyl alkane glycidyl ether is reacted with a hydroxy group or an amino group present on the carrier surface, and then, ω-alkenyl is halogenated with a halogenating agent for activation can also be exemplified. Examples of ω-alkenyl alkane glycidyl ether may include allyl glycidyl ether, 3-butenyl glycidyl ether, and 4-pentenyl glycidyl ether. Examples of a halogenating agent may include N-hlorosuccinimide, N-bromosuccinimide, and N-iodosuccinimide.

Another example of a method for introducing an active group on the carrier surface is a method for introducing an active group to a carboxy group present on the carrier surface using a condensing agent and an additive. Examples of a condensing agent may include 1-ethyl-3-(3-dimethylaminopropyl)carbodiimide (EDC), dicyclohexylcarbodiamide, and carbonyldiimidazole. Examples of an additive may include N-hydroxysuccinimide (NHS), 4-nitrophenol, and 1-hydroxybenzotriazole.

Immobilization of the AAV-binding protein of the invention on an insoluble carrier can be carried out, for example, in a buffer. Examples of a buffer may include acetic acid buffer, phosphoric acid buffer, MES (2-morpholinoethanesulfonic acid) buffer, HEPES (4-(2-hydroxyethyl)-1-piperazineethanesulfonic acid) buffer, Tris (Tris(hydroxymethyl)aminomethane) buffer, and boric acid buffer. The reaction temperature for immobilization may be set as appropriate in consideration of reactivity of an active group and stability of an AAV-binding protein. The reaction temperature at the time of immobilization may be, for example, from 5°C to 50°C, preferably from 10°C to 35°C.

The AAV adsorbent of the invention can be used, for example, by filling it into a column to purify AAV. Specifically, for example, by adding a solution containing AAV to a column filled with the AAV adsorbent of the invention, the AAV is adsorbed by the adsorbent, and the AAV adsorbed by the adsorbent is eluted such that the AAV can be purified. In other words, the present invention provides a method for purifying AAV comprising a step of adding a solution containing AAV to a column filled with the AAV adsorbent of the invention to allow the AAV to be adsorbed by the adsorbent and a step of eluting the AAV adsorbed by the adsorbent. The solution containing AAV can be added to the column using, for example, a liquid transfer means such as a pump. Adding a liquid to a column described herein is also referred to as "transferring the liquid to the column." The solution containing AAV may be treated by solvent substitution with an appropriate buffer before being added to the column. Also, the column may be equilibrated with an appropriate buffer before adding the solution containing AAV to the column. It is expected that the equilibration will allow, for example, purification of AAV to a higher degree of purity. Examples of the buffer used for solvent substitution and equilibration may include a phosphate buffer, an acetate buffer, and an MES buffer. An inorganic salt such as 10 mM to 100 mM sodium chloride may be further added to such buffer, for example. The buffer used for solvent substitution and the buffer used for equilibration may be the same or different. In addition, when components other than AAV, such as contaminants, remain on the column after the solution containing AAV is passed through the column, such components can be removed from the column before elution of the AAV adsorbed on the AAV adsorbent. Components other than AAV can be removed from the column using, for example, an appropriate buffer. As for the buffer used for removing components other than AAV, for example, the description of the buffer used for solvent substitution and equilibration can be applied mutatis mutandis. The AAV adsorbed by the AAV adsorbent can be eluted, for example, by weakening the interaction between the AAV and the ligand (AAV-binding protein of the invention). Examples of means for weakening the interaction between the AAV and the ligand (AAV-binding protein of the invention) may include pH decrease due to buffer, addition of a counterpeptide, temperature increase, and change of salt concentration. Specifically, the AAV adsorbed by the AAV adsorbent can be eluted using, for example, an appropriate eluate. Examples of the eluate include a more acidic buffer than the buffer used for solvent substitution and equilibration. Examples of such a buffer include citric acid buffer, glycine-HCl buffer, and acetate buffer. The pH of the eluate can be set, for example, within a range that does not affectthe function of AAV (e.g., binding to an antigen).

By purifying AAV in the above manner, for example, purified AAV can be obtained. In other words, the method for purifying AAV may be, in one aspect, a method for producing AAV, and specifically, a method for producing purified AAV. AAV is obtained, for example, as an elution fraction containing AAV. This means that the fraction containing the eluted AAV can be obtained. For example, the AAV fraction can be obtained by a conventional method. As a method for obtaining the AAV fraction, a method comprising replacing a collection container every fixed time or fixed capacity, a method comprising replacing a collection container according to the shape of an eluate chromatogram, and a method using an automated fraction collector such as autosampler can be exemplified. Furthermore, AAV can also be recovered from the fraction containing AAV. Recovery of AAV from the fraction containing AAV can be performed, for example, by known methods used for protein purification.

### ADVANTAGEOUS EFFECTS OF INVENTION

The adeno-associated virus (AAV)-binding protein of the invention is a protein in which an amino acid residue at a specific position in the extracellular domain of an AAV receptor is substituted with another amino acid residue. The AAV-binding protein of the invention has improved stability to heat, acid, and alkali as compared with the wild type (AAV-binding protein in which amino acid residues have not been substituted). Therefore, the AAV-binding protein of the invention is useful as an adsorbent ligand for purifying an AAV vector. In addition, an AAV adsorbent comprising an insoluble carrier and the AAV-binding protein of the invention immobilized on the carrier can purify an AAV vector contained in a solution with high purity as compared with an AAV adsorbent comprising an insoluble carrier and the wild-type AAV-binding protein immobilized on the carrier.

### BRIEF DESCRIPTION OF DRAWINGS

FIG. 1 is a graph showing the results of evaluating the binding activity of an adeno-associated virus (AAV) -binding protein to VLP2 (virus-like particles of AAV2) by an ELISA method. In the figure, (+) refers to the result when VLP2 is immobilized on the solid phase, and (-) refers to the result when VLP2 is not immobilized on the solid phase.
FIG. 2 is a graph showing a chromatographic pattern of AAV2 contained in a cell disruption solution purified by an AVR5e column. The peak near an elution time of 20 minutes was the AAV2 elution peak, and the fractions (Fr6-Fr9; 1 mL each) corresponding to the peak was recovered.
FIG. 3 is a profile showing the results of confirming the purification purity of AAV2 contained in the fractions (Fr6-Fr9) recovered in FIG. 2 by SDS-PAGE. AP in the figure is the analysis result of a solution obtained by diluting the sample before purification 100 times, and Fr6 to Fr9 are the analysis results of the respective fractions obtained in FIG. 2. VP1 to VP3 indicate the positions of the bands corresponding to the three types of capsid proteins constituting AAV.
FIG. 4 is a graph showing the results of recovering AAV2 contained in the solution using a column packed with a wild-type AAV-binding protein-immobilized gel or AVR5eHC-immobilized gel.
FIG. 5 is a graph showing the results of recovering AAV2 contained in the solution using a column packed with a wild-type AAV-binding protein-immobilized gel, AVR3HC-immobilized gel, or AVR8gHC-immobilized gel.

### EXAMPLES

Hereinafter, examples will be shown to explain the invention in more detail, but the invention is not limited to these examples.

### Example 1 Preparation of Adeno-Associated Virus (AAV) -Binding Protein Expression Vector

(1) The human codons of the nucleotide sequence encoding the amino acid residues from serine (Ser) at position 312 to aspartic acid (Asp) at position 500 corresponding to the extracellular domains 1 and 2 (PKD1 and PKD2) in the amino acid sequence of the AAV-binding protein set forth in SEQ ID NO: 1 (UniProt Accession No. Q8IZA0) were substituted with the *Escherichia coli* (*E. coli*) codons thereof. The codon-substituted nucleotide sequence is set forth in SEQ ID NO: 2.
(2) The gene set forth in SEQ ID NO: 2 was artificially totally synthesized and cloned into a plasmid (consigned to Eurofins Genomics K.K.). The prepared plasmid was named pUC-AAVR. This was used as a template to carry out PCR using oligonucleotides consisting respectively of the sequences set forth in SEQ ID NO: 3 (5'-AAT[CCATGG]GCTCTGCAGGCGAGTCGGTTC-3') and SEQ ID NO: 4 (5'-TTA[CTCGAG]TCAATGATGATGATGATGATGGTCTACCGCTTTGTTGACGG-3') as PCR primers (the square brackets in SEQ ID NO: 3 indicate the restriction enzyme NcoI recognition sequence, and the square brackets in SEQ ID NO: 4 indicate the restriction enzyme XhoI recognition sequence). Specifically, a reaction solution having the composition listed in Table 1 was prepared, the reaction solution was heat-treated at 98°C for 5 minutes, and PCR was performed by repeating 30 cycles of a reaction including a first step at 98°C for 10 seconds, a second step at 55°C for 5 seconds, and a third step at 72°C for 90 seconds.

**[Table 1]**

| Composition | Volume |
|---|---|
| Template DNA (10 ng/µL) | 1 µL |
| 10 µM PCR primer (SEQ ID NOS: 3, 33) | 2 µL |
| 10 µM PCR primer (SEQ ID NO: 4) | 2 µL |
| 5 × PrimeSTAR buffer (manufactured by Takara Bio Inc.) | 10 µL |
| 2.5 mM dNTPs | 4 µL |
| 2.5 U/µL PrimeSTAR HS (manufactured by Takara Bio Inc.) | 1 µL |
| H₂O | Up to 50 µL |

(3) The polynucleotide obtained in (2) was purified and digested with restriction enzymes NcoI and XhoI, and ligated to an expression vector pET26b (manufactured by MERCK MILLIPORE), which was preliminarily digested with restriction enzymes NcoI and XhoI. The *E*. *coli* BL21 (DE3) strain was transformed using the ligation product.
(4) After the transformant obtained in (3) was cultured in LB (Luria-Bertani) medium containing 50 µg/mL kanamycin, a vector pET-AAVRD2 expressing the extracellular domains 1 and 2 of the AAV-binding protein was extracted using a QIAprep Spin Miniprep kit (manufactured by QIAGEN).
(5) The nucleotide sequence of the polynucleotide encoding the AAV-binding protein and its surrounding region in the expression vector pET-AAVRD2 prepared in (4) was analyzed by a fully automated DNA sequencer Genetic Analyzer3500 (manufactured by Thermo Fisher Scientific). In the analysis, an oligonucleotide consisting of the sequence set forth in SEQ ID NO: 5 (5'-TAATACGACTCACTATAGGG-3') or SEQ ID NO: 6 (5'-ATGCTAGTTATTGCTCAGCGG-3') was used as a sequencing primer.

The amino acid sequence of a polypeptide expressed in the expression vector pET-AAVRD2 is set forth in SEQ ID NO: 7, and the sequence of a polynucleotide encoding the polypeptide is set forth in SEQ ID NO: 8. In SEQ ID NO: 7, the PelB signal peptide ranges from methionine (Met) at position 1 to alanine (Ala) at position 22, the extracellular domain of the AAV-binding protein (domains 1 and 2; the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219.

### Example 2 Evaluation of AAV-Binding Ability of AAV-Binding Protein

(1) The *E. coli* BL21 (DE3) strain transformed with pET-AAVRD2 prepared in Example 1 was inoculated in 3 mL of 2YT liquid medium (peptone at 16 g/L, yeast extract at 10 g/L, sodium chloride at 5 g/L) containing 50 µg/mL kanamycin and aerobically cultured with shaking overnight at 37°C, thereby performing preculture.
(2) The preculture solution of (1) in a volume of 200 µL was inoculated in 20 mL of 2YT liquid medium supplemented with 50 µg/mL kanamycin in a 100mL baffled flask and aerobically cultured with shaking at 37°C.
(3) The flask was cooled on ice 2.0 hours after the start of culture, isopropyl-β-D-thiogalactopyranoside (IPTG) was added to yield a final concentration of 0.1 mM, and shaking culture was continued aerobically overnight at 25°C.
(4) After the end of culture, the culture solution in an amount of 2 mL was collected, and cells were collected by centrifugation. A protein extract containing the expressed AAV-binding protein was prepared using the obtained cells with a 200 µL of a BugBuster Protein extraction kit (manufactured by MERCK MILLIPORE). The prepared protein extract was diluted 30-fold with 20 mM Tris-HCl buffer (pH 7.4) containing 150 mM sodium chloride.
(5) Preparation of VLP2 (virus-like particles of AAV2)
   (5-1) The *E. coli* JM109 strain was transformed using pRC2-mi342 Vector (manufactured by Takara Bio Inc.) and pHelper Vector (manufactured by Takara Bio Inc.). The obtained transformants were cultured with shaking overnight at 37°C in a 5L baffled flask containing 1 L of 2YT medium supplemented with carbenicillin to yield a concentration of 100 µg/mL each.
   (5-2) The cells were recovered by centrifuging the culture solution of (5-1). The pRC2-mi342 Vector and the pHelper Vector were prepared in large amounts from the obtained cells using Plasmid Mega Kits (manufactured by QIAGEN).
   (5-3) HEK293T cells were cultured in five T-225 flasks (manufactured by Thermo Fisher Scientific) containing 40 mL of D-MEM medium (manufactured by FUJIFILM Wako Pure Chemical Corporation) containing 10% (v/v) bovine serum. The pRC2-mi342 Vector and the pHelper Vector prepared in (5-2) were transfected into the cells using a TransIT-ViruGEN Transfection Reagent (manufactured by Takara Bio Inc.), and the cells were statically cultured for 3 days under the conditions of 5% carbon dioxide and 37°C.
   (5-4) VLP2 was obtained by collecting the cells after the culture in (5-3) and performing extraction and purification with an AAVpro Purification Kit (manufactured by Takara Bio Inc.). About 1 mL of a purified VLP2 solution was prepared from the five T-225 flasks.
(6) The binding property of the AAV-binding protein in the protein extract prepared in (4) to VLP2 prepared in (5) was evaluated by the ELISA method.
   (6-1) VLP2 prepared in (5) was diluted 200- to 1000-fold with 20 mM Tris-HCl buffer (pH 7.4) containing 150 mM sodium chloride and added to a 96-well microplate (manufactured by Thermo Fisher scientific) at 100 µL/well to be immobilized thereon (at 4°C for 18 hours). After the end of immobilization, blocking was performed with 20 mM Tris-HCl buffer (pH 7.4) containing 2% (w/v) SKIM MILK (manufactured by Becton, Dickinson and Company) and 150 mM sodium chloride.
   (6-2) After washing with washing buffer (20 mM Tris-HCl buffer (pH 7.4) containing 0.05% [w/v] Tween 20 (trade name) and 150 mM sodium chloride), a solution containing the AAV-binding protein to be evaluated for VLP2-binding activity was added such that the AAV-binding protein was reacted with VLP2 (at 30°C for 1 hour).
   (6-3) After the end of the reaction, the reaction product was washed with the washing buffer, and an Anti-6His antibody (manufactured by Bethyl Laboratories) diluted to 100 ng/mL was added at 100 µL/well.
   (6-4) After the reaction at 30°C for 1 hour, the reaction product was washed with the washing buffer, and TMB Peroxidase Substrate (manufactured by KPL) was added at 50 µL/well. Color development was stopped by adding 1M phosphoric acid at 50 µL/well. The absorbance at 450 nm was measured using a microplate reader (manufactured by Tecan Group Ltd.).

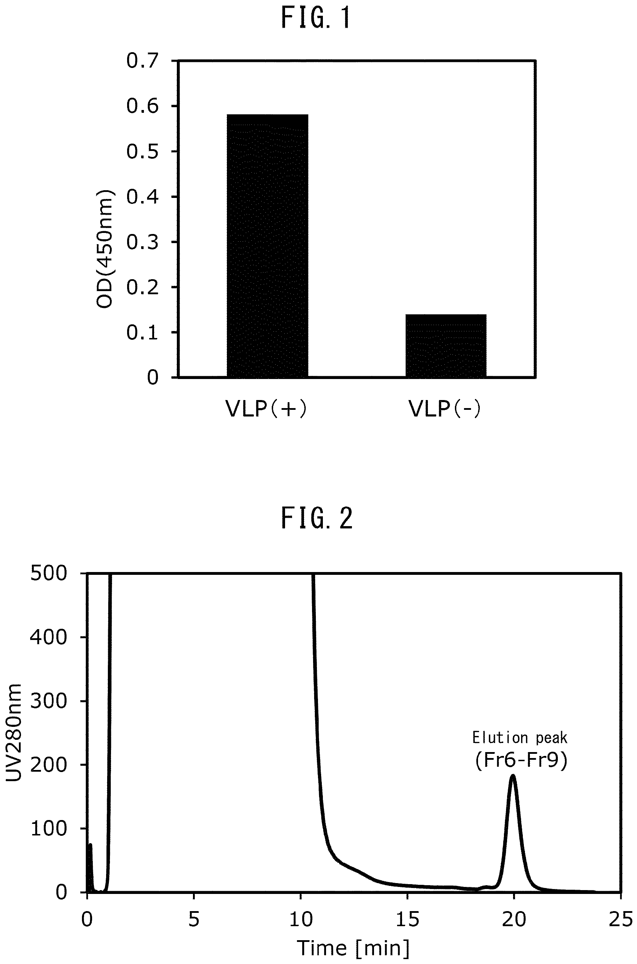

FIG. 1 shows the measurement results of the ELISA method. High absorption was observed when VLP2 was immobilized on the solid phase (VLP (+)), indicating that the AAV-binding protein expressed in this Example binds to VLP2. Since VLP2 is a virus-like particle of AAV2, this result can mean that the AAV-binding protein binds to AAV2.

### Example 3 Creation and Screening of Mutation Library of AAV-Binding Proteins (Part 1)

The polynucleotide portion encoding the AAV-binding protein in the AAV-binding protein expression vector pET-AAVRD2 was subjected to random mutagenesis by error-prone PCR.
(1) Error-prone PCR was performed using pET-AAVRD2 prepared in Example 1 as a template. After a reaction solution having the composition listed in Table 2 was prepared, the reaction solution was heat-treated at 98°C for 5 minutes, a reaction including a first step at 98°C for 30 seconds, a second step at 55°C for 20 seconds, and a third step at 72°C for 90 seconds was repeated 30 cycles, and lastly, heat treatment was performed at 72°C for 5 minutes. Thus, error-prone PCR was performed. Mutations were successfully introduced into the polynucleotide encoding the AAV-binding protein by the error-prone PCR, and the average mutation rate was 1.8 amino acid mutations per molecule.

**[Table 2]**

| Composition | Volume |
|---|---|
| Template DNA (pET-AAVRD2) (10 ng/µL) | 1 µL |
| 10 µM PCR primer (SEQ ID NO: 3) | 2 µL |
| 10 µM PCR primer (SEQ ID NO: 4) | 2 µL |
| 10 mM MnCl₂ | 1.5 µL |
| 2.5 mM dNTPs | 4 µL |
| 10 × Ex Taq Buffer (manufactured by Takara Bio Inc.) | 5 µL |
| Go Taq polymerase (manufactured by Promega Corporation) | 0.5 µL |
| H₂O | up to 50 µL |

(2) The PCR product obtained in (1) was purified and then digested with restriction enzymes NcoI and XhoI and ligated to an expression vector pET26b (manufactured by MERCK MILLIPORE), which was preliminarily digested with the same restriction enzymes.
(3) After the end of the ligation reaction, *E. coli* BL21 (DE3) was transformed with the reaction solution and cultured in LB plate medium containing 50 µg/mL kanamycin (at 37° C for 18 hours). Subsequently, the colonies formed on the plate were used as a random mutant library.
(4) The random mutant library (transformants) prepared in (3) was inoculated in 200 µL of 2YT liquid medium containing 50 µg/mL kanamycin, and cultured with shaking overnight at 37°C using a 96-well deep well plate.
(5) The culture solution prepared in (4) in an amount of 10 µL was subcultured in 500 µL of 2YT liquid medium containing 0.1 mM IPTG and 50 µg/mL kanamycin, and further cultured with shaking overnight at 25°C using a 96-well deep well plate.
(6) The culture solution of (5) was centrifuged, and the obtained culture supernatant was diluted 2-fold with 20 mM Tris-HCl buffer (pH 7.4) containing 150 mM sodium chloride. The diluted solution was heat-treated at 43°C for 15 minutes.
(7) The binding activity of AAV-binding protein to VLP2 when the heat treatment of (6) was performed and the binding activity of AAV-binding protein to VLP2 when the heat treatment of (6) was not performed were measured by the ELISA method described in Example 2 (6). The residual activity was calculated by dividing the binding activity of AAV-binding protein to VLP2 when the heat treatment was performed by the binding activity of AAV-binding protein to VLP2 when the heat treatment was not performed.
(8) About 2700 transformants were evaluated by the method of (7), and among them, transformants expressing AAV-binding proteins having improved heat stability as compared with the wild-type (without amino acid substitution) AAV-binding protein were selected. Each selected transformant was cultured, and an expression vector was prepared using a QIAprep Spin Miniprep kit (manufactured by QIAGEN).
(9) The nucleotide sequence of the polynucleotide region encoding the AAV-binding protein inserted into the obtained expression vector was analyzed by the same method as described in Example 1 (5), and the amino acid mutation site was identified.

Table 3 summarizes the amino acid substitution positions of the AAV-binding proteins expressed by the transformants selected in (8) above with respect to the wild-type (without amino acid substitution) AAV-binding protein and the residual activity (%) thereof after heat treatment. It can be said that an AAV-binding protein, in which amino acid residues from serine (Ser) at position 312 to aspartic acid (Asp) at position 500 in the amino acid sequence set forth in SEQ ID NO: 1 have at least one of the following amino acid substitutions, has improved heat stability as compared with the wild-type AAV-binding protein: Ile319Phe (this notation indicates a substitution of isoleucine at position 319 of SEQ ID NO: 1 with phenylalanine; the same applies hereinafter), Leu321Pro, Pro322Leu, Asn324Asp, Asn324Ser, Gln327Arg, Asn329Asp, Asn329Tyr, Ala330Gly, Tyr331Cys, Val332Gln, Leu333Val, Leu333Pro, Gln334Arg, Pro337Leu, Lys338Arg, Lys338Glu, Glu340Gly, Thr341Ala, Tyr342Cys, Tyr342His, Tyr342Asn, Thr343Met, Tyr344His, Asp345Asn, Trp346Leu, Trp346Arg, Gln347Leu, Gln347Pro, Leu348Pro, Ile349Thr, Thr350Met, His351Leu, Pro352Leu, Arg353Cys, Asp354Gly, Tyr355His, Tyr355Asn, Tyr355Cys, Ser356Cys, Gly357Cys, His363Arg, His363Leu, Ser364Pro, Gln365Arg, Ile366Thr, Leu367Pro, Lys368Arg, Leu369Gln, Leu369Pro, Ser370Ala, Lys371Glu, Thr373Ala, Leu376Pro, Tyr377Cys, Phe379Ser, Val381Ala, Glu384Gly, Asn387Ser, His389Gln, His389Leu, His389Arg, Gly390Ser, Glu391Lys, Tyr393Cys, Val396Ala, Lys399Glu, Lys399Arg, Arg406Ser, Val412Ala, Gln415Leu, Phe416Ser, Gln441Leu, Tyr442Phe, Lys448Arg, Glu453Gly, Lys455Arg, Glu458Gly, Ala461Ser, Ser476Arg, Leu477Pro, Asn487Asp, and Asn492Asp.

**[Table 3]**

| No. | Amino acid substitutio n | Residual activity [%] | No. | Amino acid substitution | Residual activity [%] |
|---|---|---|---|---|---|
| 1 | Asn324Asp | 46.0 | 29 | Leu333Val, Tyr342Cys | 91.8 |
| 2 | Tyr331Cys | 73.4 | 30 | Glu340Gly, Tyr342Cys | 91.3 |
| 3 | Lys338Glu | 52.6 | 31 | Tyr342His, Ile349Thr | 66.8 |
| 4 | Tyr344His | 88.2 | 32 | Tyr342Cys, Gly390Ser | 97.6 |
| 5 | Asp345Asn | 71.4 | 33 | Tyr344His, His363Arg | 82.2 |
| 6 | Trp346Leu | 85.7 | 34 | Trp346Arg, Glu458Gly | 78.1 |
| 7 | Leu348Pro | 69.6 | 35 | Gln347Leu, Thr373Ala | 85.9 |
| 8 | His351Leu | 68.7 | 36 | Tyr355His, Asn387Ser | 86.6 |
| 9 | Pro352Leu | 71.9 | 37 | Gln365Arg, His389Gln | 70.1 |
| 10 | Arg353Cys | 84.3 | 38 | Gln365Arg, Gln415Leu | 65.3 |
| 11 | Tyr355Asn | 80.6 | 39 | Ile366Thr, Gln441Leu | 65.5 |
| 12 | Ser356Cys | 84.6 | 40 | Phe379Ser, Lys455Arg | 77.4 |
| 13 | Gly357Cys | 97.0 | 41 | Glu453Gly, Ser476Arg | 56.1 |
| 14 | Gln365Arg | 57.7 | 42 | Ile319Phe, Pro322Leu, Lys448Arg | 56.6 |
| 15 | Leu367Pro | 56.9 | 43 | Leu321Pro, Tyr342Asn, Ala461Ser | 84.1 |
| 16 | Leu369Gln | 92.8 | 44 | Gln327Arg, Lys338Arg, Leu348Pro | 76.1 |
| 17 | Phe379Ser | 80.9 | 45 | Asn329Asp, Tyr342His, Tyr355Cys | 97.2 |
| 18 | Tyr393Cys | 86.4 | 46 | Asn329Asp, Ile366Thr, Lys368Arg | 49.3 |
| 19 | Val396Ala | 62.3 | 47 | Asn329Tyr, Tyr377Cys, Arg406Ser | 92.7 |
| 20 | Lys399Glu | 63.3 | 48 | Ala330Gly, Gln347Pro, Lys371Glu | 88.4 |
| 21 | Tyr442Phe | 48.7 | 49 | Tyr331Cys, Thr343Met, Tyr355Cys | 97.7 |
| 22 | Leu477Pro | 79.9 | 50 | Val332Gln, Tyr342Cys, Val412Ala | 80.3 |
| 23 | Asn487Asp | 58.3 | 51 | Leu333Pro, Pro337Leu, Thr350Met | 66.7 |
| 24 | Asn492Asp | 63.2 | 52 | Leu348Pro, Leu376Pro, Va1396A1a | 80.9 |
| 25 | Asn324Asp, Tyr355His | 776 | 53 | Asp354Gly, Leu369Gln, Lys455Arg | 90.5 |
| 26 | Asn329Tyr, Lys399Arg | 61.9 | 54 | His363Leu, Ser364Pro, His389Leu | 87.2 |
| 27 | Tyr331Cys, Leu376Pro | 74.4 | 55 | Gln365Arg, Ser370Ala, Glu391Lys | 65.7 |
| 28 | Tyr331Cys, Glu384Gly | 83.7 | 56 | Asn324Ser, Thr341Ala, Leu369Pro, Phe4l6Ser | 81.3 |
| Wild type | | 43.4 | 57 | Gln334Arg, Val381Ala, His389Arg, Tyr393Cys | 79.0 |

### Example 4 Evaluation of Thermal Stability of AAV-Binding Protein Mutants (amino acid substitution products)

Among the amino acid-substituted (mutated) AAV-binding proteins listed in Table 3, No.9(Pro352Leu substitution product), No.16 (Leu369Gln substitution product), No.29 (Leu333Val-Tyr342Cys substitution product), No.30 (Glu340Gly-Tyr342Cys substitution product), No.32 (Tyr342Cys-Gly390Ser substitution product), No.36 (Tyr355His-Asn387Ser substitution product), No.43 (Leu321Pro-Tyr342Asn-Ala461Ser substitution product), No.45 (Asn329Asp-Tyr342His-Tyr355Cys substitution product), No.47 (Asn329Tyr-Tyr377Cys-Arg406Ser substitution product), No.49 (Tyr331Cys-Thr343Met-Tyr355Cys substitution product), and No.53 (Asp354Gly-Leu369Gln-Lys455Arg substitution product) were selected, and the heat stability was evaluated again.

The nucleotide sequence and amino acid sequence of No. 9 are set forth in SEQ ID NOS: 9 and 10, respectively.
The nucleotide sequence and amino acid sequence of No. 16 are set forth in SEQ ID NOS: 11 and 12, respectively.
The nucleotide sequence and amino acid sequence of No. 29 are set forth in SEQ ID NOS: 13 and 14, respectively.
The nucleotide sequence and amino acid sequence of No. 30 are set forth in SEQ ID NOS: 15 and 16, respectively.
The nucleotide sequence and amino acid sequence of No. 32 are set forth in SEQ ID NOS: 17 and 18, respectively.
The nucleotide sequence and amino acid sequence of No. 36 are set forth in SEQ ID NOS: 19 and 20, respectively.
The nucleotide sequence and amino acid sequence of No. 43 are set forth in SEQ ID NOS: 21 and 22, respectively.
The nucleotide sequence and amino acid sequence of No. 45 are set forth in SEQ ID NOS: 23 and 24, respectively.
The nucleotide sequence and amino acid sequence of No. 47 are set forth in SEQ ID NOS: 25 and 26, respectively.
The nucleotide sequence and amino acid sequence of No. 49 are set forth in SEQ ID NOS: 27 and 28, respectively.
The nucleotide sequence and amino acid sequence of No. 53 are set forth in SEQ ID NOS: 29 and 30, respectively.

In addition, the Pro352Leu substitution in the amino acid sequence of No. 9 (SEQ ID NO: 10) is located at position 65 in SEQ ID NO: 10.
The Leu369Gln substitution in the amino acid sequence of No. 16 (SEQ ID NO: 12) is located at position 82 in SEQ ID NO: 12.
The Leu333Val and Tyr342Cys substitutions in the amino acid sequence of No. 29 (SEQ ID NO: 14) are located at positions 46 and 55 in SEQ ID NO: 14, respectively.
The Glu340Gly and Tyr342Cys substitutions in the amino acid sequence of No. 30 (SEQ ID NO: 16) are located at positions 53 and 55 in SEQ ID NO: 16, respectively.
The Tyr342Cys and Gly390Ser substitutions in the amino acid sequence of No. 32 (SEQ ID NO: 18) are located at positions 55 and 103 in SEQ ID NO: 18, respectively.
The Tyr355His and Asn387Ser substitutions in the amino acid sequence of No. 36 (SEQ ID NO: 20) are located at positions 68 and 100 in SEQ ID NO: 20, respectively.
The Leu321Pro, Tyr342Asn, and Ala461Ser substitutions in the amino acid sequence of No. 43 (SEQ ID NO: 22) are located at positions 34, 55, and 174 in SEQ ID NO: 22, respectively.
The Asn329Asp, Tyr342His, and Tyr355Cys substitutions in the amino acid sequence of No. 45 (SEQ ID NO: 24) are located at positions 42, 55, and 68 in SEQ ID NO: 24, respectively.
The Asn329Tyr, Tyr377Cys, and Arg406Ser substitutions in the amino acid sequence of No. 47 (SEQ ID NO: 26) are located at positions 42, 90, and 119 in SEQ ID NO: 26, respectively.
The Tyr331Cys, Thr343Met, and Tyr355Cys substitutions in the amino acid sequence of No. 49 (SEQ ID NO: 28) are located at positions 44, 56, and 68 in SEQ ID NO: 28, respectively.
The Asp354Gly, Leu369Gln, and Lys455Arg substitutions in the amino acid sequence of No. 53 (SEQ ID NO: 30) are located at positions 67, 82, and 168 in SEQ ID NO: 30, respectively.
(1) The transformants expressing 11 types of mutated AAV-binding proteins selected above and the transformant expressing the wild-type AAV-binding protein obtained in Example 1 (3) were each cultured by the methods described in Examples 3 (4) and (5), and then the culture solutions were centrifuged, thereby obtaining culture supernatants containing the expressed mutated AAV-binding proteins and a culture supernatant containing the wild-type AAV-binding protein.
(2) The binding activity of the AAV-binding protein in each culture supernatant obtained in (1) to VLP2 was measured using the ELISA method described in Example 2 (6). Based on the absorption at 450 nm corresponding to the above measurement results, each culture supernatant obtained in (1) was diluted with 20 mM Tris buffer (pH 7.4) containing 150 mM sodium chloride such that the measured values would be the same.
(3) The diluted protein solution was divided into three portions, and two of them were heat-treated at 50.3°C or 66.1°C for 15 minutes using a thermal cycler (manufactured by Eppendorf SE) while the remaining one was not heat-treated.
(4) The binding activity between the heat-treated or non-heat treated AAV-binding activity protein of (3) and VLP2 was measured by the ELISA method described in Example 2 (6), and the residual activity was calculated by dividing the absorbance at 450 nm with heat treatment by the absorbance at 450 nm without heat treatment.

Table 4 shows the results. It was confirmed that all of the mutated AAV-binding proteins had higher residual activity as compared with the wild-type AAV-binding protein, and therefore, the heat stability of the mutated AAV-binding protein was improved.

**[Table 4]**

| AAV-binding protein | | Residual activity [%] | |
|---|---|---|---|
| No. | Amino acid substitution | 50.3°C | 66.1°C |
| 9 | Pro352Leu | 49.0 | 46.5 |
| 16 | Leu369Gln | 32.0 | 20.3 |
| 29 | Leu333Val, Tyr342Cys | 66.7 | 54.5 |
| 30 | Glu340Gly, Tyr342Cys | 65.4 | 52.4 |
| 32 | Tyr342Cys, Gly390Ser | 76.0 | 70.5 |
| 36 | Tyr355His, Asn387Ser | 74.5 | 51.0 |
| 43 | Leu321Pro, Tyr342Asn, Ala461Ser | 55.6 | 56.3 |
| 45 | Asn329Asp, Tyr342His, Tyr355Cys | 66.0 | 46.7 |
| 47 | Asn329Tyr, Tyr377Cys, Arg406Ser | 64.5 | 58.7 |
| 49 | Tyr331Cys, Thr343Met, Tyr355Cys | 77.3 | 57.3 |
| 53 | Asp354Gly, Leu369Gln, Lys455Arg | 29.8 | 23.5 |
| Wild type | | 16.1 | 9.6 |

### Example 5 Evaluation of Acid Stability of Mutated AAV-Binding Protein (Part 1)

(1) The transformants expressing the mutated AAV-binding proteins evaluated in Example 4 (i.e., mutated AAV-binding proteins according to Nos. 9, 16, 29, 30, 32, 36, 43, 45, 47, 49, and 53 in Table 3) and the transformant expressing the wild-type AAV-binding protein obtained in Example 1 (3) were each cultured by the methods described in Examples 3 (4) and (5), and then the culture solutions were centrifuged, thereby obtaining culture supernatants containing the expressed mutated AAV-binding proteins or a culture supernatant containing the wild-type AAV-binding protein.
(2) The binding activity of the AAV-binding protein in each culture supernatant obtained in (1) to VLP2 was measured using the ELISA method described in Example 2 (6). Based on the absorption at 450 nm corresponding to the above measurement results, each culture supernatant obtained in (1) was diluted with pure water such that the measured values would be the same.
(3) The diluted protein solution was divided into two portions, one of which was mixed with an equal amount of 0.1 M glycine-HCl buffer (pH 2.5). After standing still at 30°C for 2 hours and 24 hours, the pH was adjusted to around 6 by mixing the solution with 0.5 M MES buffer (pH 6.0) at a ratio of 3:2, and the binding activity of the protein to VLP2 was measured by the ELISA method described in Example 2 (6). The other one was not acid-treated, and the dilution ratio was the same as the condition of the acid treatment case. The pH was adjusted to around 6 by mixing the solution with 0.5 M MES buffer (pH 6.0) at a ratio of 3:2, and the binding activity of the protein to VLP2 was measured by the ELISA method described in Example 2 (6).
(4) The residual activity was calculated by dividing the absorbance at 450 nm with the acid treatment described in (3) by the absorbance at 450 nm without the acid treatment.

Table 5 shows the results. In all the mutated AAV-binding proteins evaluated in Example 4, the residual activity after treatment for 2 hours and 24 hours under the conditions of 30°C and pH 2.5 was improved as compared with the wild type. In other words, it was confirmed that the acid stability of the mutated AAV-binding proteins was improved. In particular, No.30 (Glu340Gly-Tyr342Cys substitution product, SEA ID NO: 16), No.32 (Tyr342Cys-Gly390Ser substitution product, SEQ ID NO:18), and No.45 (Asn329Asp-Tyr342His-Tyr355Cys substitution product, SEQ ID NO:24) had significantly improved acid stability as compared with the wild-type AAV-binding protein.

**[Table 5]**

| AAV-binding protein | | PH2.5 Residual activity [%] | |
|---|---|---|---|
| No. | Amino acid substitution | 2h | 24h |
| 9 | Pro352Leu | 17.6 | 5.6 |
| 16 | Leu369Gln | 17.3 | 10.0 |
| 29 | Leu333Val, Tyr342Cys | 52.2 | 40.2 |
| 30 | Glu340Gly, Tyr342Cys | 70.5 | 56.1 |
| 32 | Tyr342Cys, Gly390Ser | 70.7 | 64.7 |
| 36 | Tyr355His, Asn387Ser | 16.1 | 8.9 |
| 43 | Leu321Pro, Tyr342Asn, Ala461Ser | 41.4 | 28.3 |
| 45 | Asn329Asp, Tyr342His, Tyr355Cys | 64.4 | 47.6 |
| 47 | Asn329Tyr, Tyr377Cys, Arg406Ser | 43.6 | 34.8 |
| 49 | Tyr331Cys, Thr343Met, Tyr355Cys | 52.8 | 16.6 |
| 53 | Asp354Gly, Leu369Gln, Lys455Arg | 18.2 | 13.2 |
| Wild type | | 7.9 | 5.1 |

### Example 6 Creation and Screening of Mutation Library of AAV-Binding Proteins (Part 2)

Among the mutated AAV-binding proteins evaluated in Examples 4 and 5, No. 32 (Tyr342Cys-Gly390Ser substitution product, SEQ ID NO: 18) was selected (named AVR2). The polynucleotide portion encoding the protein was subjected to random mutagenesis by error-prone PCR.
(1) Using the plasmid pET-AVR2 into which the polynucleotide (SEQ ID NO: 17) encoding AVR2 (SEQ ID NO: 18) was inserted as a template, a reaction solution having the composition listed in Table 6 was prepared, and then error-prone PCR was performed under the same temperature conditions as in Example 3 (1). Mutations were successfully introduced into the polynucleotide encoding AVR2 by the error-prone PCR, and the average mutation rate was 1.5 amino acid mutations per molecule.

**[Table 6]**

| Composition | Volume |
|---|---|
| 10 ng/µL template DNA | 1 µL |
| 10 µM PCR primer (SEQ ID NO: 3) | 2 µL |
| 10 µM PCR primer (SEQ ID NO: 4) | 2 µL |
| 10 mM MnCl₂ | 0.5 µL |
| 2.5 mM dNTPs | 4 µL |
| 10 × Ex Taq Buffer (manufactured by Takara Bio Inc.) | 5 µL |
| Go Taq polymerase (manufactured by Promega Corporation) | 0.5 µL |
| H₂O | up to 50 µL |

(2) The PCR product obtained in (1) was purified and then digested with restriction enzymes NcoI and XhoI and ligated to an expression vector pET26b (manufactured by MERCK MILLIPORE), which was preliminarily digested with the same restriction enzymes.
(3) After the end of the ligation reaction, *E. coli* BL21 (DE3) was transformed with the reaction solution and cultured in LB plate medium containing 50 µg/mL kanamycin (at 37°C overnight). Subsequently, the colonies formed on the plate were used as a random mutant library.
(4) The random mutant library (transformants) prepared in (3) cultured by the methods described in Example 3 (4) and (5).
(5) The culture solution of (4) was centrifuged, and the obtained culture supernatant was diluted 4-fold with ultrapure water. The diluted culture solution in an amount of 60 µL and 60 µL of 0.1 M glycine-HCl buffer (pH 3.0) were mixed, and heat treatment and acid treatment were performed at 51.1°C for 15 minutes.
(6) The binding activity of AAV-binding protein to VLP2 when the heat treatment of (5) was performed and the binding activity of AAV-binding protein to VLP2 when the heat treatment of (5) was not performed were measured by the ELISA method described in Example 2 (6). The residual activity was calculated by dividing the binding activity of AAV-binding protein to VLP2 when the heat treatment was performed by the binding activity of AAV-binding protein to VLP2 when the heat treatment was not performed.
(7) A random mutant library of about 1800 transformants was evaluated by the method of (6), and among them, transformants expressing AAV-binding proteins having improved residual activity as compared with the parent molecule AVR2 were selected. Each selected transformant was cultured, and an expression vector was prepared using a QIAprep Spin Miniprep kit (manufactured by QIAGEN).
(8) The nucleotide sequence of the polynucleotide region encoding the AAV-binding protein inserted into the obtained expression vector was analyzed by the method described in Example 1 (5), and the amino acid mutation site was identified.

Table 7 summarizes the amino acid substitution positions of the AAV-binding proteins expressed by the transformants selected in (7) above with respect to AVR2 (Tyr342Cys-Gly390Ser substitution product) and the residual activity (%) thereof after heat treatment and acid treatment.

It can be said that an AAV-binding protein, in which amino acid residues from serine (Ser) at position 312 to aspartic acid (Asp) at position 500 in the amino acid sequence set forth in SEQ ID NO: 1 have at least one of the following amino acid substitutions, has improved heat stability and acid stability as compared with the wild-type AAV-binding protein: Ile319Phe, Ile319Asn, Ile319Ser, Thr320Ile, Leu321Pro, Lys323Glu, Leu328Gln, Leu328Pro, Tyr331His, Val332Ala, Val332Glu, Leu333Pro, Pro336Gln, Pro337Gln, Lys338Asn, Tyr(Cys)342Arg (this notation indicates a substitution of tyrosine at position 342 of SEQ ID NO: 1 with cysteine and then further by arginine.; the same applies hereinafter), Thr350Ser, Gln365Arg, Ile366Phe, Phe379Ser, Val383Ala, Gln386Arg, His389Asp, Gly392Cys, Val394Ala, Val396Ala, Lys399Glu, Ile409Val, Ser476Gly, Ser476Arg, Asn487Asp, Thr490Ser, and Asn492Asp.

**[Table 7]**

| No. | Amino acid substitution | Residual activity [%] | No. | Amino acid substitution | Residual activity [%] |
|---|---|---|---|---|---|
| 58 | Ile319Phe | 73.9 | 71 | His389Asp | 71.9 |
| 59 | Ile319Asn | 26.1 | 72 | Val394Ala | 32.0 |
| 60 | Lys323Glu | 80.2 | 73 | Val396Ala | 48.9 |
| 61 | Leu328Gln | 43.5 | 74 | Ser476Gly | 63.4 |
| 62 | Leu328Pro | 38.8 | 75 | Ser476Arg | 96.3 |
| 63 | Tyr331His | 43.1 | 76 | Asn492Asp | 36.4 |
| 64 | Val332Ala | 41.4 | 77 | Leu321Pro, Ile409Val | 34.3 |
| 65 | Va1332Glu | 29.4 | 78 | Leu328Pro, Ile366Phe | 55.9 |
| 66 | Leu333Pro | 31.8 | 79 | Lys338Asn, Asn487Asp | 73.1 |
| 67 | Pro337Gln | 45.6 | 80 | Gln365Arg, Gly392Cys | 49.9 |
| 68 | Tyr(Cys)342Arg | 70.4 | 81 | Phe379Ser, Ser476Gly | 66.3 |
| 69 | Thr350Ser | 49.9 | 82 | Ser476Arg, Thr490Ser | 70.6 |
| 70 | Val383Ala | 32.8 | 83 | Ile319Ser, Thr320Ile, Lys399Glu | 70.2 |
| 32 | AVR2 | 25.5 | 84 | Pro336Gln, Gln386Arg, Asn487Asp | 48.4 |

### Example 7 Integration of Stabilized Amino Acid Substitutions (Part 1)

By integrating the amino acid substitutions involved in the improvement of heat stability and acid stability of the AAV-binding proteins found in Example 6 in AVR2 (SEQ ID NO: 18), the stability was further improved. Specifically, among the amino acid-substituted (mutated) AAV-binding proteins listed in Table 7, No. 75 (integrated amino acid substitutions of Ser476Arg for AVR2) was selected (named AVR3). The amino acid substitutions described in any one of the following (a) to (d) were integrated into the protein. The Ser476Arg substitution in the amino acid sequence of AVR3 (SEQ ID NO: 32) is located at position 189 in SEQ ID NO: 32.
(a) Ile319Phe (named AVR4a)
(b) Asn487Asp (named AVR4d)
(c) Ile319Phe and Lys323Glu (named AVR5b)
(d) Lys399Glu and Asn487Asp (named AVR5e)

Hereinafter, a method for producing the following AAV-binding proteins will be described in detail.

### (a) AVR4a

From the amino acid substitutions clarified in Example 6 that are involved in improving the stability to heat and acid, Ile319Phe was selected and integrated in AVR3 (SEQ ID NO: 32), thereby preparing AVR4a. Specifically, AVR4a was prepared by introducing a mutation that yields Ile319Phe in the polynucleotide (SEQ ID NO: 31) encoding AVR3 (SEQ ID NO: 32).

(a-1) A reaction solution having the composition listed in Table 1 was prepared using the plasmid pET-AVR3 into which the polynucleotide (SEQ ID NO: 31) encoding AVR3 was inserted as a template and oligonucleotides consisting respectively of the sequences set forth in SEQ ID NO: 4 and SEQ ID NO: 33 (5'-CCATGGGCTCTGCAGGCGAGTCGGTTCAGTTTACCC-3') as PCR primers. The reaction solution was heat-treated at 98°C for 5 minutes, a reaction including a first step at 98°C for 10 seconds, a second step at 55°C for 5 seconds, and a third step at 72°C for 1 minute was repeated 30 cycles, and lastly, heat treatment was performed at 72°C for 5 minutes. Thus, PCR was performed.

(a-2) The amplified PCR product was subjected to agarose gel electrophoresis and purified from the gel using a QIAquick Gel Extraction kit (manufactured by QIAGEN). The purified PCR product was named 4ap.

(a-3) 4ap obtained in (a-2) was purified and digested with restriction enzymes NcoI and XhoI, and ligated to an expression vector pET26b (manufactured by MERCK MILLIPORE), which was preliminarily digested with restriction enzymes NcoI and XhoI. The *E. coli* BL21 (DE3) strain was transformed using the ligation product.

(a-4) The transformant obtained in (a-3) was cultured in LB medium supplemented with 50 µg/mL kanamycin. Plasmids were extracted from the collected bacterial cells (transformants), thereby obtaining a plasmid pET-AVR4a containing a polynucleotide encoding AVR4a with four amino acid substitutions in the wild-type AAV-binding protein.

(a-5) The nucleotide sequence of pET-AVR4a was analyzed in the same manner as in Example 1 (5).

The amino acid sequence of AVR4a with a signal sequence and a polyhistidine tag is set forth in SEQ ID NO: 34, and the sequence of the polynucleotide encoding AVR4a is set forth in SEQ ID NO: 35. In SEQ ID NO: 34, the PelB signal peptide ranges from methionine (Met) at position 1 to alanine (Ala) at position 22, the AAV-binding protein AVR4a (domains 1 and 2 corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 34, phenylalanine of Ile319Phe is present at position 32, cysteine of Tyr342Cys is present at position 55, serine of Gly390Ser is present at position 103, and arginine of Ser476Arg is present at position 189.

### (b) AVR4d

From the amino acid substitutions clarified in Example 6 that are involved in improving the stability to heat and acid, Asn487Asp was selected and accumulated in AVR3 (SEQ ID NO: 32), thereby preparing AVR4d. Specifically, AVR4d was prepared by introducing a mutation that causes Asn487Asp in the polynucleotide (SEQ ID NO: 31) encoding AVR3 (SEQ ID NO: 32).

(b-1) A reaction solution having the composition listed in Table 8 was prepared using pET-AVR3 as a template and oligonucleotides consisting respectively of the sequences set forth in SEQ ID NO: 5 and SEQ ID NO: 36 (5'-GATTCTGATGGCGCAACCGACTCCACCACC-3') as PCR primers. The reaction solution was heat-treated at 98°C for 5 minutes, a reaction including a first step at 98°C for 10 seconds, a second step at 55°C for 5 seconds, and a third step at 72°C for 1 minute was repeated 30 cycles, and lastly, heat treatment was performed at 72°C for 5 minutes. Thus, PCR was performed.

**[Table 8]**

| Composition | Volume |
|---|---|
| 10 ng/µL template DNA | 1 µL |
| 10 µM PCR primer (SEQ ID NOS: 5, 37, 40, 44) | 1 µL |
| 10 µM PCR primer (SEQ ID NOS: 36, 6, 41, 45) | 1 µL |
| 5 × PrimeSTAR Buffer (manufactured by Takara Bio Inc.) | 10 µL |
| 2.5 mM dNTPs | 4 µL |
| 2.5 U/µL PrimeSTAR HS (manufactured by Takara Bio Inc.) | 1 µL |
| H₂O | up to 50 µL |

(b-2) The amplified PCR product was purified by the same method as in (a-2). The purified PCR product was named 4dF.

(b-3) PCR was performed in the same manner as in (b-1) and (b-2) except that pET-AVR3 was used as a template and oligonucleotides consisting respectively of the sequences set forth in SEQ ID NO: 6 and SEQ ID NO: 37 (5'-GGTGGTGGAGTCGGTTGCGCCATCAGAATC-3') were used as PCR primers. The purified PCR product was named 4dR.

(b-4) The two types of PCR products (4dF, 4dR) obtained in (b-2) and (b-3) were mixed, thereby preparing a reaction solution having the composition listed in Table 9. The reaction solution was heat-treated at 98°C for 5 minutes, and PCR was performed by a reaction of 30 cycles of a first step at 98°C for 10 seconds, a second step at 55°C for 5 seconds, and a third step at 72°C for 1 minute, thereby obtaining a PCR product 4dp in which 4dF and 4dR were joined to each other.

**[Table 9]**

| Composition | Volume |
|---|---|
| PCR product (4dF) | 1 µL |
| PCR product (4dR) | 1 µL |
| 10 µM PCR primer (SEQ ID NO: 5) | 1 µL |
| 10 µM PCR primer (SEQ ID NO: 6) | 1 µL |
| 5 × PrimeSTAR Buffer (manufactured by Takara Bio Inc.) | 10 µL |
| 2.5 mM dNTPs | 4 µL |
| 2.5 U/µL PrimeSTAR HS (manufactured by Takara Bio Inc.) | 1 µL |
| H₂O | up to 50 µL |

(b-5) The PCR product 4dp obtained in (b-4) was treated in the same manner as in (a-3) and (a-4), thereby obtaining a plasmid pET-AVR4d containing a polynucleotide encoding AVR4d with four amino acid substitutions in the wild-type AAV-binding protein.

(b-6) The nucleotide sequence of pET-AVR4d was analyzed in the same manner as in Example 1 (5).

The amino acid sequence of AVR4d with a signal sequence and a polyhistidine tag is set forth in SEQ ID NO: 38, and the sequence of the polynucleotide encoding AVR4d is set forth in SEQ ID NO: 39. In SEQ ID NO: 38, the PelB signal peptide ranges from methionine (Met) at position 1 to alanine (Ala) at position 22, the AAV-binding protein AVR4d (corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 38, cysteine of Tyr342Cys is present at position 55, serine of Gly390Ser is present at position 103, arginine of Ser476Arg is present at position 189, and aspartic acid of Asn487Asp is present at position 200.

### (c) AVR5b

From the amino acid substitutions clarified in Example 6 that are involved in improving the stability to heat and acid, Ile319Phe and Lys323Glu were selected and integrated in AVR3 (SEQ ID NO: 32), thereby preparing AVR5b. Specifically, AVR5b was prepared by introducing mutations that yield Ile319Phe and Lys323Glu in the polynucleotide (SEQ ID NO: 31) encoding AVR3 (SEQ ID NO: 32).

(c-1) A reaction solution having the composition listed in Table 8 was prepared using pET-AVR3 as a template and oligonucleotides consisting respectively of the sequences set forth in SEQ ID NO: 40 (5'-TTTT[GGTCTC]AGTTCTCCGGCAGGGTAACTGAACCGAC-3') and SEQ ID NO: 41 (5'-TTTT[GGTCTC]AGAACGAAGTACAACTGAATGCGTATGTG-3') (both the square brackets in SEQ ID NO: 40 and the square brackets in SEQ ID NO: 41 indicate the restriction enzyme BsaI recognition sequence) as PCR primers. The reaction solution was heat-treated at 98°C for 5 minutes, a reaction including a first step at 98°C for 10 seconds, a second step at 55°C for 5 seconds, and a third step at 72°C for 6 minutes was repeated 30 cycles, and lastly, heat treatment was performed at 72°C for 5 minutes. Thus, PCR was performed.

(c-2) The amplified PCR product was purified by the same method as in (a-2). The purified PCR product was named 5bp.

(C-3) The 5bp obtained in (c-2) was treated with the restriction enzyme DpnI (manufactured by NEB) at 37°C for 1.5 hours, and then treated at 80°C for 20 minutes. The thus obtained gene was subjected to agarose gel electrophoresis and purified from the gel using a QIAquick Gel Extraction kit (manufactured by QIAGEN).

(c-4) The DpnI-treated product obtained in (c-3) was digested with a restriction enzyme BsaI (manufactured by NEB) under the reaction solution having the composition listed in Table 10 and ligated with T4DNA ligase (manufactured by NEB).

**[Table 10]**

| Composition | Volume |
|---|---|
| PCR product (20 ng/µL) | 5 µL |
| 10 × CutSmart Buffer (manufactured by NEB) | 1.5 µL |
| BsaI (manufactured by NEB) | 1 µL |
| T4 DNA ligase buffer (manufactured by NEB) | 1.5 µL |
| T4 DNA ligase (manufactured by NEB) | 1 µL |
| H₂O | up to 15 µL |

(c-5) The *E. coli* BL21 (DE3) strain was transformed using the ligation product of (c-4). The obtained transformant was cultured in LB medium supplemented with 50 µg/mL kanamycin. Plasmids were extracted from the collected bacterial cells (transformants), thereby obtaining a plasmid pET-AVR5b containing a polynucleotide encoding AVR5b with five amino acid substitutions in the wild-type AAV-binding protein.

(c-6) The nucleotide sequence of pET-AVR5b was analyzed in the same manner as in Example 1 (5).

The amino acid sequence of AVR5b with a signal sequence and a polyhistidine tag is set forth in SEQ ID NO: 42, and the sequence of the polynucleotide encoding AVR5b is set forth in SEQ ID NO: 43. In SEQ ID NO: 42, the PelB signal peptide ranges from methionine (Met) at position 1 to alanine (Ala) at position 22, the AAV-binding protein AVR5b (corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 42, phenylalanine of Ile319Phe is present at position 32, glutamic acid of Lys323Glu is present at position 36, cysteine of Tyr342Cys is present at position 55, serine of Gly390Ser is present at position 103, and arginine of Ser476Arg is present at position 189.

### (d) AVR5e

From the amino acid substitutions clarified in Example 6 that are involved in improving the stability to heat and acid, Lys399Glu and Asn487Asp were selected and integrated in AVR3 (SEQ ID NO: 32), thereby preparing AVR5e. Specifically, AVR5e was prepared by introducing a mutation that yields Lys399Glu in the polynucleotide (SEQ ID NO: 39) encoding AVR4d (SEQ ID NO: 38) prepared in (b).

(d -1) A plasmid pET-AVR5e containing the polynucleotide encoding AVR5e with five amino acid substitutions in the wild-type AAV-binding protein was obtained in the same manner as in (c-1) to (c-5) except that pET-AVR4d obtained in (b-5) was used as a template and oligonucleotides consisting respectively of the sequences set forth in SEQ ID NO: 44 (5'-TTTT[GGTCTC]ACTTGCGTGGCTCCGGTTCCACGGTAACG-3') and SEQ ID NO: 45 (5'-TTTT[GGTCTC]ACAAGAACCGTCCTCCGATCGCCATTG-3') (both the square brackets in SEQ ID NO: 44 and the square brackets in SEQ ID NO: 45 indicate the restriction enzyme BsaI recognition sequence) were used as PCR primers.

(d-2) The nucleotide sequence of pET-AVR5e was analyzed in the same manner as in Example 1 (5).

The amino acid sequence of AVR5e with a signal sequence and a polyhistidine tag is set forth in SEQ ID NO: 46, and the sequence of the polynucleotide encoding AVR5e is set forth in SEQ ID NO: 47. In SEQ ID NO: 46, the PelB signal peptide ranges from methionine (Met) at position 1 to alanine (Ala) at position 22, the AAV-binding protein AVR5e (corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 46, cysteine of Tyr342Cys is present at position 55, serine of Gly390Ser is present at position 103, glutamic acid of Lys399Glu is present at position 112, arginine of Ser476Arg is present at position 189, and aspartic acid of Asn487Asp is present at position 200.

### Example 8 Evaluation of Acid Stability of Mutated AAV-Binding Protein (Part 2)

(1) The transformants expressing the mutated AAV-binding protein AVR3 obtained in Example 6 and the mutated AAV-binding proteins (AVR4a, AVR4d, AVR5b, and AVR5e) prepared in Example 7 were each cultured by the methods described in Examples 3 (4) and (5), and then the culture solutions were centrifuged, thereby obtaining culture supernatants containing the expressed mutated AAV-binding proteins.
(2) The binding activity of the mutated AAV-binding protein in each culture supernatant obtained in (1) to VLP2 was measured using the ELISA method described in Example 2 (6). Based on the absorption at 450 nm corresponding to the above measurement results, each culture supernatant obtained in (1) was diluted with pure water such that the measured values would be the same.
(3) Each diluted mutated AAV-binding protein solution was divided into four portions, and each portion was mixed with an equal amount of 0.1 M glycine-HCl buffer (pH 3.0). Three of the four samples were heat-treated at 51.5°C, 60.1°C, and 66.1°C, respectively, for 15 minutes, and the remaining one was left to stand still at room temperature (25°C) for 15 minutes. Thereafter, the pH was adjusted to around 6 by mixing each sample with 0.5 M MES buffer (pH 6.0) at a ratio of 3:2, and the binding activity of the protein to VLP2 was measured by the ELISA method described in Example 2 (6).
(4) The residual activity was calculated by dividing the absorbance at 450 nm when the heat treatment and acid treatment described in (3) were performed by the absorbance at 450 nm when standing still was performed at room temperature.

Table 11 shows the results. Each of the mutated AAV-binding proteins (AVR3, AVR4a, AVR4d, AVR5b, and AVR5e) prepared in Example 7 had a residual activity higher than that of AVR3 (No. 75 in Table 7) regardless of the treatment temperature. This clarifies that the five mutated AAV-binding proteins have significantly improved stability to heat treatment under acidic conditions, i.e., stability to acid and heat, as compared with the wild-type AAV-binding protein.

**[Table 11]**

| AAV-binding protein | | | Residual activity after heat trea tment [%] | | |
|---|---|---|---|---|---|
| SEQ ID NO | Name | Amino acid substitution | 51.5°C | 60.1°C | 66.1°C |
| 34 | AVR4a | Ile319Phe, Tyr342Cys, Gly390Ser, Ser476Arg | 57 | 16 | 8 |
| 38 | AVR4d | Tyr342Cys, Gly390Ser, Ser476Arg, Asn487Asp | 66 | 35 | 18 |
| 42 | AVR5b | Ile319Phe, Lys323Glu, Tyr342Cys, Gly390Ser, Ser476Arg | 87 | 43 | 23 |
| 46 | AVR5e | Tyr342Cys, Gly390Ser, Lys399Glu, Ser476Arg, Asn487Asp | 77 | 56 | 43 |
| 32 | AVR3 | Tyr342Cys, Gly390Ser, Ser476Arg | 21 | 3 | 2 |

### Example 9 Preparation of Mutated AAV-Binding Protein with Improved C-terminus

(1) A reaction solution having the composition listed in Table 12 was prepared using pET-AVR5e prepared in Example 7 (d) as a template and oligonucleotides consisting respectively of the sequences set forth in SEQ ID NO: 3 and SEQ ID NO: 48 (5'-AT[CTCGAG]TCATCCGCAGGTATCGTTGCGGCAATGATGATGATGATGATGGTCTAC -3') (the square brackets in SEQ ID NO: 48 indicate the restriction enzyme XhoI recognition sequence) as PCR primers. The reaction solution was heat-treated at 94°C for 2 minutes, a reaction including a first step at 98°C for 10 seconds, a second step at 52°C for 30 seconds, and a third step at 68°C for 1.5 minutes was repeated 25 cycles. Thus, PCR was performed.

**[Table 12]**

| Composition | Volume |
|---|---|
| 10 ng/µL template | 1 µL |
| 10 µM PCR primer (SEQ ID NOS: 3, 57, 61, 67, 71, 72, 74) | 1.5 µL |
| 10 µM PCR primer (SEQ ID NOS: 48, 58, 62, 68, 73, 75) | 1.5 µL |
| 10 × KOD Buffer (manufactured by TOYOBO CO., LTD.) | 5 µL |
| 2 mM dNTPs | 5 µL |
| 25 mM MgSO₄ | 3 µL |
| 1 U/µL KOD Plus (manufactured by TOYOBO CO., LTD.) | 1 µL |
| H₂O | up to 50 µL |

(1) The PCR product obtained in (1) was subjected to agarose gel electrophoresis, purified from the gel using a QIAquick Gel Extraction kit (manufactured by QIAGEN), and digested with restriction enzymes NcoI and XhoI, and ligated to an expression vector pET26b (manufactured by MERCK MILLIPORE), which was preliminarily digested with restriction enzymes NcoI and XhoI. The *E. coli* BL21 (DE3) strain was transformed using the ligation product.

(3) After the obtained transformant was cultured in LB medium containing 50 µg/mL kanamycin, a plasmid pET-AVR5eHC encoding a protein (named AVR5eHC) having a tag for immobilization at the C-terminus of AVR5e was obtained using a QIAprep Spin Miniprep kit (manufactured by QIAGEN).

(4) The nucleotide sequence of pET-AVR5eHC obtained was analyzed in the same manner as in Example 1 (5).

The nucleotide sequence and amino acid sequence of AVR5eHC are set forth in SEQ ID NOS: 49 and 50, respectively. In SEQ ID NO: 50, the PelB signal peptide ranges from methionine (Met) at position 1 to alanine (Ala) at position 22, the AAV-binding protein AVR5e ranges from serine (Ser) at position 25 to aspartic acid (Asp) at position 213, the histidine tag ranges from histidine (His) at position 214 to histidine (His) at position 219, and the cysteine tag sequence as a tag for immobilization ranges from cysteine (Cys) at position 220 to glycine (Gly) at position 226.

### Example 10 Mass-Preparation of Mutated AAV-Binding Protein with Improved C-terminus

(1) A transformant capable of expressing AVR5eHC obtained by transforming the *E. coli* BL21 (DE3) strain with pET-AVR5eHC prepared in Example 9 (3) was inoculated in a 200-mL baffled flask containing 40 mL of liquid medium (Phytone Peptone at 16 g/L, yeast extract at 10 g/L, sodium chloride at 5 g/L) containing 50 µg/mL kanamycin and aerobically cultured with shaking at 37°C for 10 hours, thereby performing preculture.
(2) After inoculating 10 mL of the preculture solution of (1) in a 3-L fermenter (manufactured by Biott Corporation) into which 0.9 L of liquid medium containing glucose at 20 g/L, yeast extract at 40 g/L, trisodium phosphate dodecahydrate at 6 g/L, disodium hydrogen phosphate dodecahydrate at 18 g/L, ammonium chloride at 2 g/L, magnesium sulfate at 2 g/L, iron sulphate at 2 g/L, manganese chloride at 10 g/L, and kanamycin sulfate at 50 mg/L was introduced, the main culture was started under the conditions of a preset temperature of 30°C, a pH of 6.9 to 7.1, and an airflow rate of 3 VVM. For pH control, 50% (v/v) phosphoric acid is used as acid and 14% (w/v) ammonia water is used as alkali, and for the control of dissolved oxygen, the number of rotations of stirring was changed in the range of a lower limit of 500 rpm/an upper limit of 1000 rpm. At a time point when the glucose concentration became unmeasurable after the start of culture, a feed medium (glucose at 425 g/L, yeast extract at 140 g/L, magnesium sulfate heptahydrate at 12 g/L) was added while controlling dissolved oxygen.
(3) When the absorbance at 600 nm reached about 100, the preset temperature was controlled to be lowered to 25°C. After confirming that the temperature reached the thus set temperature, 1.1 mL of 0.5 mM IPTG was added, and culture was continued at 25°C. Culture was terminated about 42 hours after the start of culture, and the culture solution was centrifuged at 4°C and 8000 rpm for 20 minutes, thereby collecting bacterial cells.
(4) The bacterial cells collected in (3) were suspended in a 20 mM Tris-HCl buffer (pH 7.4) containing 0.5M sodium chloride and 20 mM imidazole to yield a concentration of 5 mL/1 g (of cells), and then the cells were disrupted using an ultrasonic generator (Insonator 201M [manufactured by KUBOTA Corporation co., ltd.]) at 8°C and an output power of about 150 W for about 10 minutes. A solution of the disrupted cells was centrifuged twice at 4°C and 8000 rpm for 20 minutes, thereby collecting the supernatant.
(5) The supernatant obtained in (4) was applied to an XK26/20 column column (manufactured by Cytiva) filled with 50 mL of Ni Sepharose 6 Fast Flow (manufactured by Cytiva) which was preliminarily equilibrated with Tris-HCl buffer (pH 7.4) containing 0.5 M sodium chloride and 20 mM imidazole (hereinafter, also referred to as "equilibrium solution A"). After washing with the equilibration solution A, elution was performed with 20 mM Tris-HCl buffer (pH 7.4) containing 0.5 M imidazole and 150 mM sodium chloride.
(6) The eluate obtained in (5) was dialyzed against 20 mM Tris buffer (pH 7.4) containing 4 mM imidazole and 150 mM sodium chloride, and then applied to an XK26/20 column column (manufactured by Cytiva) filled with 50 mL of TALON Metal affinity Resin (manufactured by Takara Bio Inc.) equilibrated with the same buffer solution. After washing with the buffer used for equilibration, elution was performed with 20 mM Tris-HCl buffer (pH 7.4) containing 0.5 M imidazole and 150 mM sodium chloride.
(7) The eluate obtained in (6) was dialyzed against 20 mM Tris-HCl buffer (pH 7.4) containing 150 mM sodium chloride, thereby preparing about 200 mg of AVR5eHC protein.

### Example 11 Preparation of Viral Vector (AAV2-EGFP)

(1) A nucleotide sequence (SEQ ID NO: 52) was designed, in which a restriction enzyme EcoRI recognition sequence (GAATTC) was added to the 5' end side of a polynucleotide encoding an enhanced green fluorescent protein (EGFP) consisting of the amino acid sequence set forth in SEQ ID NO: 51, and a stop codon (TAG) and a BamHI recognition sequence (GGATTC) were added to the 3' end side of the polynucleotide.
(2) The polynucleotide consisting of the sequence set forth in SEQ ID NO: 52 was totally synthesized and cloned into a plasmid (consigned to FASMAC, named pUC-EGFP). The *E*. *coli* JM109 strain was transformed with pUC-EGFP, and the obtained transformant was cultured. pUC-EGFP was extracted from the culture solution using a QIAprep Spin Miniprep kit (manufactured by QIAGEN).
(3) pUC-EGFP obtained in (2) was digested with restriction enzymes EcoRI and BamHI, and ligated to an expression vector pAAV-CMV (manufactured by Takara Bio Inc.), which was preliminarily digested with restriction enzymes EcoRI and BamHI. The *E*. *coli* JM109 strain was transformed using the ligation product. After the obtained transformant was cultured in LB medium containing 100 µg/mL carbenicillin, an expression vector pAAV-EGFP expressing EGFP was extracted using a QIAprep Spin Miniprep kit (manufactured by QIAGEN).
(4) The transformant obtained in (3) was cultured with shaking overnight at 37°C in a 5L baffled flask containing 1 L of 2YT medium supplemented with 100 µg/mL carbenicillin. After the end of culture, the cells were collected by centrifugation. A large amount of pAAV-EGFP was prepared from the recovered cells using a Plasmid Mega Kit (manufactured by QIAGEN).
(5) Preparation of AAV2-EGFP
   (5-1) The *E. coli* JM109 strain was transformed using pRC2-mi342 Vector (manufactured by Takara Bio Inc.) and pHelper Vector (manufactured by Takara Bio Inc.). Large amounts of pRC2-mi342 and pHelper were prepared by performing the same operation as in (4) using the obtained transformant.

(5-2) HEK293T cells were cultured in ten T-225 flasks (manufactured by Thermo Fisher Scientific) containing 45 mL of D-MEM medium (manufactured by FUJIFILM Wako Pure Chemical Corporation) containing 10% (v/v) bovine serum. Transfection was carried out by adding a complex of pAAV-EGFP prepared in (4), pRC2-mi342 and pHelper prepared in (5-1), and polyethyleneimine (manufactured by Polysciences Inc.) thereto, and the cells were statically cultured for 3 days under the conditions of 5% (v/v) carbon dioxide and 37°C. After the culture, the detached cells were recovered by centrifugation and each set of cells obtained from five T-225 flasks were cryopreserved at -80°C.

(5-3) The frozen cells obtained in (5-2) were thawed and suspended in 10 mL of 20 mM Tris-HCl buffer (pH 7.4) containing 0.5 M sodium chloride, 4 mM magnesium chloride, and 0.01% (w/v) Tween 20 (trade name). One-twentieth (1/20) the amount of Benzonase (manufactured by MERCK MILLIPORE) was added thereto, and the mixture was left to stand sill at 37°C for 1 hour and then centrifuged at 13000 × g at 4°C for 10 minutes, thereby obtaining a supernatant. Ammonium sulfate was added to the obtained supernatant so as to result in 15% saturation, and the mixture was centrifuged again under the same conditions. The obtained supernatant was applied to a filter having a pore size of 0.45 µm, and suspended matter was removed.

(5-4) The supernatant from which suspended matter was removed was applied to a 5-mL AVB Sepharose column (manufactured by Cytiva) which was preliminarily equilibrated with 20 mM Tris-HCl buffer (pH 8.0) containing 0.5 M sodium chloride (hereinafter, also referred to as "equilibrium solution B"). After washing with the equilibration solution B, elution was performed with 0.1M acetate buffer (pH 2.5) containing 0.5 M sodium chloride. The obtained eluate was neutralized by adding one-fourth (1/4) the amount of 1 M Tris-HCl buffer (pH 8.5) containing 20 mM magnesium chloride, thereby obtaining a solution of AAV2-EGFP which is an AAV vector. The AAV2-EGFP concentration in the solution was quantified by qPCR using an AAVpro Titration Kit (manufactured by Takara Bio Inc.).

### Example 12 Preparation of AAV-Binding Protein-Immobilized Gel (Part 1)

(1) Gel into which a maleimide group was introduced was prepared by chemically modifying the hydroxy group on the surface of a hydrophilic vinyl polymer for a separating agent (manufactured by Tosoh Corporation: TOYOPEARL). To 3.5 g of the prepared gel, 28 mg of the AVR5eHC protein prepared in Example 10 and Tris(2-carboxyethyl)phosphine (TCEP) with a final concentration of 0.1 mM as a reducing agent were added. The reaction was carried out by shaking at pH 7.4 and 4°C for 15 hours. As a result, gel on which AVR5e was immobilized (named AVR5e-immobilized gel) was prepared.
(2) A Tricorn 10/50 column (manufactured by Cytiva) was filled with 2 mL of the AVR5e-immobilized gel prepared in (1), thereby preparing a column (named AVR5e column). The AVR5e column was connected to AKTAprime plus (manufactured by Cytiva) and equilibrated with 50 mM phosphate buffer (pH 7.4) (hereinafter also referred to as "equilibrium solution C") containing 150 mM sodium chloride and 0.05% (w/v) Tween 20 (trade name), and then 10 mL of the cell extract containing AAV2-EGFP obtained in Example 11 (5-3) was applied thereto at a flow rate of 1 mL/min.
(3) After washing with the equilibration solution C, AAV2-EGFP was eluted with 0.1M acetate buffer (pH 2.5) containing 0.5 M sodium chloride from the AVR5e column. The eluate was fractionated in 1 ml aliquots and collected as fractions. The recovered fractions were neutralized by adding 250 µL of 1 M Tris-HCl buffer (pH 8.5) containing 20 mM magnesium chloride.
(4) SDS-PAGE and silver staining using a Pierce Silver Stain Kit (manufactured by Thermo Fisher Scientific) were carried out to compare the purity of the eluate obtained in (3) with the purity of the cell extract obtained in Example 10 (5-3), which was a sample before purification.

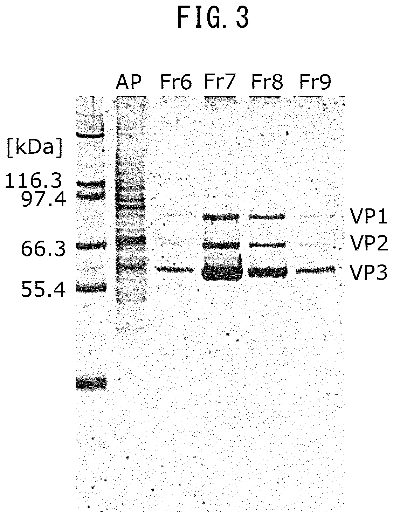

The chromatographic pattern for the AVR5e column obtained in (3) is shown in FIG. 2, and the results of SDS-PAGE in (4) are shown in FIG. 3. The peak of AAV2-EGFP appeared around an elution time of 20 minutes (FIG. 2). When fractions (Fr6 to Fr9) corresponding to the peak were subjected to SDS-PAGE, only the bands corresponding to VP1, VP2, and VP3 constituting AAV were confirmed (FIG. 3). On the other hand, innumerable bands derived from contaminants were confirmed for the cell extract applied to the AVR5e column, in addition to the above bands (FIG. 3). The above results clarify that AAV can be purified with high purity by an AAV adsorbent containing gel and the AAV-binding protein of the invention immobilized on the gel.

### Example 13 Creation and Screening of Mutation Library of AAV-Binding Proteins (Part 3)

Among the mutated AAV-binding proteins evaluated in Example 7, AVR5e (Tyr342Cys-Gly390Ser-Lys399Glu-Ser476Arg-Asn487Asp substitution product, SEQ ID NO: 46) was selected. The polynucleotide portion encoding the protein was subjected to random mutagenesis by error-prone PCR.
(1) Using the plasmid pET-AVR5e into which the polynucleotide (SEQ ID NO: 47) encoding AVR5e (SEQ ID NO: 46) was inserted as a template, a reaction solution having the composition listed in Table 6 was prepared, and then error-prone PCR was performed under the same temperature conditions as in Example 3 (1). Mutations were successfully introduced into the polynucleotide encoding AVR5e by the error-prone PCR, and the average mutation rate was 1.7 amino acid mutations per molecule.
(2) After purifying the PCR product obtained in (1), a random mutant library was prepared by the methods described in Examples 6 (2) and (3). The library (transformants) was cultured by the methods described in Example 3 (4) and (5).
(3) The culture solution of (2) was centrifuged, and the obtained culture supernatant was diluted 32-fold with ultrapure water. The diluted culture solution in an amount of 60 µL and 60 µL of 0.1 M glycine hydrochloric acid buffer (pH 3.0) were mixed, and heat treatment and acid treatment were performed at 61.0°C for 15 minutes.
(4) The binding activity of AAV-binding protein to VLP2 when the treatment of (3) was performed and the binding activity of AAV-binding protein to VLP2 when the treatment of (3) was not performed were measured by the ELISA method described in Example 2 (6). The residual activity was calculated by dividing the binding activity of AAV-binding protein to VLP2 when the heat treatment was performed by the binding activity of AAV-binding protein to VLP2 when the heat treatment was not performed.
(5) A random mutant library of about 1800 transformants was evaluated by the method of (4), and among them, transformants expressing AAV-binding proteins having improved residual activity as compared with the parent molecule AVR5e were selected. Each selected transformant was cultured, and an expression vector was prepared using a QIAprep Spin Miniprep kit (manufactured by QIAGEN).
(6) The nucleotide sequence of the polynucleotide region encoding the AAV-binding protein inserted into the obtained expression vector was analyzed by the method described in Example 1 (5), and the amino acid mutation site was identified.

Table 13 summarizes the amino acid substitution positions of the AAV-binding proteins expressed by the transformants selected in (5) above with respect to AVR5e (Tyr342Cys-Gly390Ser-Lys399Glu-Ser476Arg-Asn487Asp substitution product) and the residual activity (%) thereof after heat treatment and acid treatment.

It can be said that an AAV-binding protein, in which amino acid residues from serine (Ser) at position 312 to aspartic acid (Asp) at position 500 in the amino acid sequence set forth in SEQ ID NO: 1 have at least one of the following amino acid substitutions, has improved heat stability and acid stability as compared with the wild-type AAV-binding protein: Val317Asp, Gln318Pro, Thr320Ala, Asn324Asp, Val326Ala, Val326Glu, Leu328Pro, Asn329His, Val332Ala, Lys362Glu, Lys362Asn, Lys371Glu, Lys371Asn, Leu376Pro, Glu378Gly, Glu378Val, Phe379Ser, Phe379Tyr, Val381Asp, Gly392Cys, Thr397Ser, Glu401Val, Arg406His, Asn492Asp, and Val499Glu.

**[Table 13]**

| No. | Amino acid substitution | Residual activity [%] | No. | Amino acid substitution | Residual activity [%] |
|---|---|---|---|---|---|
| 85 | Val317Asp | 50.2 | 94 | Val326Glu, Asn329His | 44.9 |
| 86 | Lys371Glu | 41.6 | 95 | Val326Ala, Lys362Glu | 45.3 |
| 87 | Lys371Asn | 56.5 | 96 | Val326Glu, Phe379Ser | 60.9 |
| 88 | Leu376Pro | 41.7 | 97 | Val332Ala, Lys371Glu | 49.6 |
| 89 | Glu378Gly | 46.1 | 98 | Leu376Pro, Asn492Asp | 47.6 |
| 90 | Gly392Cys | 53.3 | 99 | Gln318Pro, Phe379Tyr, Glu401Val | 54.0 |
| 91 | Val317Asp, Lys371Asn | 55.7 | 100 | Asn324Asp, Lys362Glu, Val499Glu | 50.1 |
| 92 | Thr320Ala, Val381Asp | 52.4 | 101 | Val326Glu, Asn329His, Glu378Val | 48.5 |
| 93 | Asn324Asp, Lys362Glu | 50.7 | 102 | Leu328Pro, Lys362Asn, Thr397Ser, Arg406His | 41.1 |
| AVR5e | | 25.5 | | | |

### Example 14 Integration of Stabilized Amino Acid Substitutions (Part 2)

By integrating the amino acid substitutions involved in the improvement of heat stability and acid stability of the AAV-binding proteins found in Example 13 in AVR5e (SEQ ID NO: 46), the stability was further improved. Specifically, among the amino acid-substituted (mutated) AAV-binding proteins listed in Table 13, No. 93 (integrated amino acid substitutions of Asn324Asp and Lys362Glu for AVR5e) (named AVR7a) and No.91 (integrated amino acid substitutions of Val317Asp and Lys371Asn for AVR5e) (named AVR7b) were selected. The amino acid substitutions described in the following (a) or (b) were integrated into the proteins. The Asn324Asp and Lys362Glu substitutions in the amino acid sequence of AVR7a (SEQ ID NO: 53) are located at positions 37 and 75 in SEQ ID NO: 53, respectively. In addition, the Val317Asp and Lys371Asn substitutions in the amino acid sequence of AVR7b (SEQ ID NO: 55) are located at positions 30 and 84 in SEQ ID NO: 55, respectively.
(a) AVR7a with Val317Asp introduced therein (named AVR8a)
(b) AVR7b with Lys362Glu introduced therein (named AVR8g)

Hereinafter, a method for producing the following AAV-binding proteins will be described in detail.

### (a) AVR8a

From the amino acid substitutions clarified in Example 13 that are involved in improving the stability to heat and acid, Val317Asp was selected and integrated in AVR7a (SEQ ID NO: 53), thereby preparing AVR8a. Specifically, AVR8a was prepared by introducing a mutation that yields Val317Asp in the polynucleotide (SEQ ID NO: 54) encoding AVR7a (SEQ ID NO: 53).

(a-1) A reaction solution having the composition listed in Table 12 was prepared using a plasmid pET-AVR7a into which the polynucleotide (SEQ ID NO: 54) encoding AVR7a was inserted as a template and oligonucleotides consisting respectively of the sequences set forth in SEQ ID NO: 57 (5'-TTTT[GGTCTC]AGCAGGCGAGTCGGATCAGATTACCC-3') and SEQ ID NO: 58 (5'-TTTT[GGTCTC]ACTGCAGAGCCCATGGCCATCGCCGG-3') (both the square brackets in SEQ ID NO: 57 and the square brackets in SEQ ID NO: 58 indicate the restriction enzyme BsaI recognition sequence) as PCR primers. The reaction solution was heat-treated at 98°C for 5 minutes, a reaction including a first step at 98°C for 10 seconds, a second step at 55°C for 5 seconds, and a third step at 72°C for 6 minutes was repeated 30 cycles, and lastly, heat treatment was performed at 72°C for 5 minutes. Thus, PCR was performed.

(a-2) The amplified PCR product was subjected to agarose gel electrophoresis and purified from the gel using a QIAquick Gel Extraction kit (manufactured by QIAGEN). The purified PCR product was named 8ap.

(a-3) The 8ap obtained in (a-2) was treated with the restriction enzyme DpnI (manufactured by NEB) at 37°C for 1.5 hours, and then treated at 80°C for 20 minutes. The thus obtained gene was subjected to agarose gel electrophoresis and purified from the gel using a QIAquick Gel Extraction kit (manufactured by QIAGEN).

(a-4) The DpnI-treated product obtained in (a-3) was digested with a restriction enzyme BsaI (manufactured by NEB) under the reaction solution having the composition listed in Table 10 and ligated with T4DNA ligase (manufactured by NEB).

(a-5) The *E. coli* BL21 (DE3) strain was transformed using the ligation product of (a-4). The obtained transformant was cultured in LB medium supplemented with 50 µg/mL kanamycin. Plasmids were extracted from the collected bacterial cells (transformants), thereby obtaining a plasmid pET-AVR8a containing a polynucleotide encoding AVR8a with eight amino acid substitutions in the wild-type AAV-binding protein.

(a-6) The nucleotide sequence of pET-AVR8a was analyzed in the same manner as in Example 1 (5).

The amino acid sequence of AVR8a with a signal sequence and a polyhistidine tag is set forth in SEQ ID NO: 59, and the sequence of the polynucleotide encoding AVR8a is set forth in SEQ ID NO: 60. In SEQ ID NO: 59, the PelB signal peptide ranges from methionine (Met) at position 1 to alanine (Ala) at position 22, the AAV-binding protein AVR8a (domains 1 and 2 corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 59, aspartic acid of Val317Asp is present at position 30, aspartic acid of Asn324Asp is present at position 37, cysteine of Tyr342Cys is present at position 55, glutamic acid of Lys362Glu is present at position 75, serine of Gly390Ser is present at position 103, glutamic acid of Lys399Glu is present at position 112, arginine of Ser476Arg is present at position 189, and aspartic acid of Asn487Asp is present at position 200.

### (b) AVR8g

From the amino acid substitutions clarified in Example 13 that are involved in improving the stability to heat and acid, Lys362Glu was selected and accumulated in AVR7b (SEQ ID NO: 55), thereby preparing AVR8g. Specifically, AVR8g was prepared by introducing a mutation that yields Lys362Glu in the polynucleotide (SEQ ID NO: 56) encoding AVR7b (SEQ ID NO: 55).

(b -1) A plasmid pET-AVR8g containing the polynucleotide encoding AVR8g with eight amino acid substitutions in the wild-type AAV-binding protein was obtained in the same manner as in (a-1) to (a-5) except that a plasmid pET-AVR7b into which the polynucleotide (SEQ ID NO: 56) encoding AVR7b was inserted was used as a template and oligonucleotides consisting respectively of the sequences set forth in SEQ ID NO: 61 (5'-TTTT[GGTCTC]ACGAGATGGAAGGAGAACATTCGCAG-3') and SEQ ID NO: 62 (5'-TTTT[GGTCTC]ACTCGCCGCTGTAGTCGCGAGG-3') (both the square brackets in SEQ ID NO: 61 and the square brackets in SEQ ID NO: 62 indicate the restriction enzyme BsaI recognition sequence) were used as PCR primers.

(b-2) The nucleotide sequence of pET-AVR8g was analyzed in the same manner as in Example 1 (5).

The amino acid sequence of AVR8g with a signal sequence and a polyhistidine tag is set forth in SEQ ID NO: 63, and the sequence of the polynucleotide encoding AVR8g is set forth in SEQ ID NO: 64. In SEQ ID NO: 63, the PelB signal peptide ranges from methionine (Met) at position 1 to alanine (Ala) at position 22, the AAV-binding protein AVR8g (domains 1 and 2 corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 63, aspartic acid of Val317Asp is present at position 30, cysteine of Tyr342Cys is present at position 55, glutamic acid of Lys362Glu is present at position 75, aspartic acid of Lys371Asn is present at position 84, serine of Gly390Ser is present at position 103, glutamic acid of Lys399Glu is present at position 112, arginine of Ser476Arg is present at position 189, and aspartic acid of Asn487Asp is present at position 200.

### Example 15 Evaluation of Acid Stability of Mutated AAV-Binding Protein (Part 3)

(1) The transformants expressing the mutated AAV-binding protein AVR7a obtained in Example 13 and the mutated AAV-binding proteins (AVR8a and AVR8g) prepared in Example 14 were each cultured by the methods described in Examples 3 (4) and (5), and then the culture solutions were centrifuged, thereby obtaining culture supernatants containing the expressed mutated AAV-binding proteins.
(2) The binding activity of the mutated AAV-binding protein in each culture supernatant obtained in (1) to VLP2 was measured using the ELISA method described in Example 2 (6). Based on the absorption at 450 nm corresponding to the above measurement results, each culture supernatant obtained in (1) was diluted with pure water such that the measured values would be the same.
(3) Each diluted mutated AAV-binding protein solution was divided into three portions, and each portion was mixed with an equal amount of 0.1 M glycine-HCl buffer (pH 3.0). Two of the three samples were heat-treated at 80.8°C and 89.8°C, respectively, for 15 minutes, and the remaining one was left to stand still at room temperature (25°C) for 15 minutes. Thereafter, the pH was adjusted to around 6 by mixing each sample with 0.5 M MES buffer (pH 6.0) at a ratio of 3:2, and the binding activity of the protein to VLP2 was measured by the ELISA method described in Example 2 (6).
(4) The residual activity was calculated by dividing the absorbance at 450 nm when the heat treatment and acid treatment described in (3) were performed by the absorbance at 450 nm when standing still was performed at room temperature.

Table 14 shows the results. Each of the mutated AAV-binding proteins (AVR8a and AVR8g) prepared in Example 14 had a residual activity higher than that of AVR7a (No. 93 in Table 14) when treated at 80.8°C. This clarifies that the two mutated AAV-binding proteins have significantly improved stability to heat treatment under acidic conditions, i.e., stability to acid and heat, as compared with the wild-type AAV-binding protein.

**[Table 14**

| AAV-binding protein | | | Residual activity after heat treatme nt [%] | |
|---|---|---|---|---|
| SEQ ID NO | Name | Amino acid substitution | 80.8°C | 89.8°C |
| 59 | AVR8a | Val317Asp, Asn324Asp, Tyr342Cys, Lys362Glu, Gly390S er, Lys399Glu, Ser476Arg, Asn487Asp | 66 | 52 |
| 63 | AVR8g | Va1317Asp, Tyr342Cys, Lys362Glu, Lys371Asn, Gly390S er, Lys399Glu, Ser476Arg, Asn487Asp | 67 | 55 |
| 53 | AVR7a | Asn324Asp, Tyr342Cys, Lys362Glu, Gly390Ser, Lys399G lu, Ser476Arg, Asn487Asp | 57 | 54 |

### Example 16 Creation and Screening of Mutation Library of AAV-Binding Proteins (Part 4)

Among the mutated AAV-binding proteins evaluated in Example 15, AVR8g (Val317Asp-Tyr342Cys-Lys362Glu-Lys371Asn-Gly390Ser-Lys399Glu-Ser476Arg-Asn487Asp substitution product, SEQ ID NO: 63) was selected. The polynucleotide portion encoding the protein was subjected to random mutagenesis by error-prone PCR.
(1) Using the plasmid pET-AVR8g into which the polynucleotide (SEQ ID NO: 64) encoding AVR8g (SEQ ID NO: 63) was inserted as a template, a reaction solution having the composition listed in Table 6 was prepared, and then error-prone PCR was performed under the same temperature conditions as in Example 3 (1). Mutations were successfully introduced into the polynucleotide encoding AVR8g by the error-prone PCR, and the average mutation rate was 2.3 amino acid mutations per molecule.
(2) After purifying the PCR product obtained in (1), a random mutant library was prepared by the methods described in Examples 6 (2) and (3). The library (transformants) was cultured by the methods described in Example 3 (4) and (5).
(3) The culture solution of (2) was centrifuged, and the obtained culture supernatant was diluted 16-fold with ultrapure water. The diluted culture solution in an amount of 60 µL and 60 µL of 0.1 M glycine-HCl buffer (pH 10.0) were mixed, and heat treatment and acid treatment were performed at 41.2°C for 15 minutes.
(4) The binding activity of AAV-binding protein to VLP2 when the treatment of (3) was performed and the binding activity of AAV-binding protein to VLP2 when the treatment of (3) was not performed were measured by the ELISA method described in Example 2 (6). The residual activity was calculated by dividing the binding activity of AAV-binding protein to VLP2 when the heat treatment was performed by the binding activity of AAV-binding protein to VLP2 when the heat treatment was not performed.
(5) A random mutant library of about 1800 transformants was evaluated by the method of (4), and among them, transformants expressing AAV-binding proteins having improved residual activity as compared with the parent molecule AVR8g were selected. Each selected transformant was cultured, and an expression vector was prepared using a QIAprep Spin Miniprep kit (manufactured by QIAGEN).
(6) The nucleotide sequence of the polynucleotide region encoding the AAV-binding protein inserted into the obtained expression vector was analyzed by the method described in Example 1 (5), and the amino acid mutation site was identified.
(7) Table 15 summarizes the amino acid substitution positions of the AAV-binding proteins expressed by the transformants selected in (6) above with respect to AVR8g (Val317Asp-Tyr342Cys-Lys362Glu-Lys371Asn-Gly390Ser-Lys399Glu-Ser476Arg-Asn487Asp substitution product) and the residual activity (%) thereof after heat treatment and acid treatment.

It can be said that an AAV-binding protein, in which amino acid residues from serine (Ser) at position 312 to aspartic acid (Asp) at position 500 in the amino acid sequence set forth in SEQ ID NO: 1 have at least one of the following amino acid substitutions, has improved heat stability and alkali stability as compared with the wild-type AAV-binding protein: Ser312Pro, Ala313Ser, Lys323Arg, Asn324Ser, Gln327His, Gln327Leu, Asn329Ile, Glu335Gly, Glu335Val, Glu340Val, Thr341Pro, Thr343Ser, Tyr344Phe, Trp346Cys, Arg353Cys, Tyr355Cys, Ser356Cys, Gly357Cys, Met359Leu, Glu360Lys, Glu360Val, Gly361Cys, Lys(Glu)362Gly, Ser364Leu, Ile366Thr, Leu369Gln, Lys(Asn)371Asp, Leu372Pro, Leu372Gln, Thr373Ala, Pro374Leu, Leu376Pro, Glu378Gly, Glu378Val, Phe379Cys, Phe379Ser, Lys380Glu, Val381Ala, Ile382Val, Val383Ala, Glu384Val, Gln386Arg, His389Arg, Tyr393Cys, Val394Asp, Val394Ile, Asn395Ser, Val396Ala, Arg403His, Gln415Arg, Thr426Ala, Gln432Leu, Gln432Arg, Gln441Arg, His443Leu, Lys448Glu, Ile456Val, Asp483Asn, Ser488Leu, and Val499Ile.

**[Table 15]**

| No. | Amino acid substitution | Residual activity [%] | No. | Amino acid substitution | Residual activity [%] |
|---|---|---|---|---|---|
| 103 | Glu340Val | 35.0 | 128 | Ser312Pro, Pro374Leu | 74.9 |
| 104 | Tyr344Phe | 40.3 | 129 | Ala313Ser, Glu378Gly | 67.5 |
| 105 | Arg353Cys | 62.1 | 130 | Lys323Arg, Glu384Val | 63.1 |
| 106 | Tyr355Cys | 63.8. | 131 | Asn324Ser, Gly357Cys | 70.3 |
| 107 | Ser356Cys | 67.6 | 132 | Gln327His, Glu378Val | 56.5 |
| 108 | Met359Leu | 32.4 | 133 | Gln327Leu, Lys(Asn)371Asp | 62.1 |
| 109 | Glu360Lys | 40.2 | 134 | Asn329Ile, Val499Ile | 63.9 |
| 110 | Glu360Val | 33.3 | 135 | Glu335Gly, Gln386Arg | 36.3 |
| 111 | Lys(Glu)362Gly | 41.1 | 136 | Glu335Val, Thr343Ser | 38.8 |
| 112 | Ser364Leu | 45.3 | 137 | Thr341Pro, Phe379Ser | 86.3 |
| 113 | Ile366Thr | 41.4 | 138 | Gly361Cys, Gly(Ser)390Gly | 51.5 |
| 114 | Leu372Pro | 91.6 | 139 | Leu369Gln, Pro374Leu | 85.2 |
| 115 | Leu372Gln | 72.8 | 140 | Leu372Pro, Val396Ala | 92.8 |
| 116 | Thr373Ala | 57.8 | 141 | Leu376Pro, Gly(Ser)390Gly | 85.0 |
| 117 | Leu376Pro | 76.7 | 142 | Glu378Gly, Arg403His | 76.6 |
| 118 | Glu378Gly | 66.7 | 143 | Val381Ala, Ile382Val | 90.3 |
| 119 | Phe379Ser | 24.3 | 144 | Val383Ala, Gln415Arg | 65.7 |
| 120 | Lys380Glu | 59.5 | 145 | Trp346Cys, Arg353Cys, His389Arg | 63.5 |
| 121 | Val38 1 Ala | 89.9 | 146 | Leu372Gln, Leu376Pro, Val394Asp | 81.4 |
| 122 | Gln386Arg | 34.9 | 147 | Phe379Cys, Gln432Leu, Ser488Leu | 79.7 |
| 123 | Tyr393Cys, | 60.5 | 148 | Glu384Val, Gln432Arg, Ile456Val | 37.4 |
| 124 | Gln441 Arg | 25.2 | 149 | Val394Ile, Asn395Ser, Thr426Ala | 70.5 |
| 125 | His443Leu | 36.5 | 150 | Val396Ala, Gln441Arg, Lys448Glu | 49.7 |
| 126 | Lys448Glu | 34.5 | 63 | AVR8G | 23.1 |
| 127 | Asp483Asn | 23.3 | | | |

### Example 17 Monomerization of Mutated AAV-Binding Protein (Part 1)

Among the mutated AAV-binding proteins found to have improved heat stability and alkali stability in Example 16, No.143 (integrated amino acid substitutions of Val381Ala and Ile382Val for AVR8g) (named AVR10) was selected. An amino acid substitution of Tyr(Cys)342Ser was introduced therein to monomerize the protein (named AVR10s). Specifically, AVR10s was prepared by introducing a mutation that yields Tyr(Cys)342Ser in the polynucleotide (SEQ ID NO: 66) encoding AVR10 (SEQ ID NO: 65). The Val381Ala and Ile382Val substitutions in the amino acid sequence of AVR10 (SEQ ID NO: 65) are located at positions 94 and 95 in SEQ ID NO: 65, respectively.
(1) A plasmid pET-AVR10s containing the polynucleotide encoding AVR10s with ten amino acid substitutions in the wild-type AAV-binding protein was obtained in the same manner as in Example 14 (a-1) to (a-5) except that pET26b-AVR10 into which the polynucleotide (SEQ ID NO: 66) encoding AVR10 was inserted was used as a template and oligonucleotides consisting respectively of the sequences set forth in SEQ ID NO: 67 (5'-TTTT[GGTCTC]AGAAACCTCTACGTATGACTG-3') and SEQ ID NO: 68 (5'-TTTT[GGTCTC]ATTTCCCCTTTCGGTGG-3') (both the square brackets in SEQ ID NO: 67 and the square brackets in SEQ ID NO: 68 indicate the restriction enzyme BsaI recognition sequence) were used as PCR primers.
(2) The nucleotide sequence of pET-AVR10s was analyzed in the same manner as in Example 1 (5).

The amino acid sequence of AVR10s with a signal sequence and a polyhistidine tag is set forth in SEQ ID NO: 69, and the sequence of the polynucleotide encoding AVR10s is set forth in SEQ ID NO: 70. In SEQ ID NO: 69, the PelB signal peptide ranges from methionine (Met) at position 1 to alanine (Ala) at position 22, the AAV-binding protein AVR10s (corresponding to the domain ranging from positions 312 to 500 of SEQ ID NO: 1) ranges from serine (Ser) at position 25 to aspartic acid (Asp) at position 213, and the tag sequence ranges from histidine (His) at position 214 to histidine (His) at position 219. In SEQ ID NO: 69, aspartic acid of Val317Asp is present at position 30, serine of Tyr(Cys)342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200.

### Example 18 Monomerization of Mutated AAV-Binding Protein (Part 2)

A mutated AAV binding protein was prepared, into which an amino acid substitution of Tyr(Cys)342Met was introduced to monomerize the mutated AAV binding protein AAV5eHC prepared in Example 9, and to which PKD3 (amino acid residues ranging from tyrosine at position 501 to lysine at position 597 in SEQ ID NO: 1) was further added (named AVR5eMHCD3). Specifically, AVR5eMHCD3 was prepared by introducing a mutation that yields Tyr(Cys)342Met in the polynucleotide (SEQ ID NO: 49) encoding AVR5eHC (SEQ ID NO: 50) and then adding PKD3 thereto.
(1) A plasmid pET-AVR5eMHC containing the polynucleotide encoding the mutated AAV-binding protein in which an amino acid substitution of Tyr(Cys)342Met was introduced into AVR5eHC was obtained in the same manner as in Example 14 (a-1) to (a-5) except that pET26b-AVR5eHC was used as a template and oligonucleotides consisting respectively of the sequences set forth in SEQ ID NO: 71 (5'-TTTT[GGTCTC]AGAAACCATGACGTATGACTG-3') and SEQ ID NO: 68 (both the square brackets in SEQ ID NO: 71 and the square brackets in SEQ ID NO: 68 indicate the restriction enzyme BsaI recognition sequence) were used as PCR primers.
(2) PCR was performed in the same manner as in Example 14 (a-1) except that pUC-AAVR was used as a template and oligonucleotides consisting respectively of the sequences set forth in SEQ ID NO: 72 (5'-TTTT[GGTCTC]AGATGTTTGTTATTTTCGGGCTGAACAATC-3') and SEQ ID NO: 73 (5'-TTTT[GGTCTC]AAGACTATCCGCCGGTAGCGAACGC-3') (both the square brackets in SEQ ID NO: 72 and the square brackets in SEQ ID NO: 73 indicate the restriction enzyme BsaI recognition sequence) were used as PCR primers.
(3) PCR was performed in the same manner as in Example 14 (a-1) except that pET26b-AVR5eMHC prepared in (1) was used as a template and oligonucleotides consisting respectively of the sequences set forth in SEQ ID NO: 74 (5'-TTTT[GGTCTC]AGTCTACCGCTTTGTTG-3') and SEQ ID NO: 75 (5'-TTTT[GGTCTC]ACATCATCATCATCATCAT-3') (both the square brackets in SEQ ID NO: 74 and the square brackets in SEQ ID NO: 75 indicate the restriction enzyme BsaI recognition sequence) were used as PCR primers.
(4) The PCR products amplified in (2) and (3) were each subjected to agarose gel electrophoresis and purified from the gel using a QIAquick Gel Extraction kit (manufactured by QIAGEN). The purified PCR product of (2) was named D3p, and the PCR product of (3) was named 5MVp.
(5) D3p and 5Mvp obtained in (4) were treated with the restriction enzyme DpnI (manufactured by NEB) at 37°C for 1.5 hours, and then treated at 80°C for 20 minutes. The thus obtained genes were each subjected to agarose gel electrophoresis and purified from the gel using a QIAquick Gel Extraction kit (manufactured by QIAGEN).
(6) The DpnI-treated product obtained in (5) was digested with a restriction enzyme BsaI (manufactured by NEB) under the reaction solution having the composition listed in Table 16 and ligated with T4DNA ligase (manufactured by NEB).

**[Table 16]**

| Composition | Volume |
|---|---|
| 20 ng/µL PCR product (D3p) | 5 µL |
| 20 ng/µL PCR product (5MVp) | 5 µL |
| 10 × CutSmart buffer (manufactured by NEB) | 1.5 µL |
| BsaI (manufactured by NEB) | 1 µL |
| T4 DHA ligase buffer (manufactured by NEB) | 1.5 µL |
| T4 DHA ligase (manufactured by NEB) | 1 µL |
| H₂O | up to 15 µL |

(7) The *E. coli* BL21 (DE3) strain was transformed using the ligation product of (6). The obtained transformant was cultured in LB medium supplemented with 50 µg/mL kanamycin. Plasmids were extracted from the collected bacterial cells (transformants), thereby obtaining a plasmid pET-AVR5eMHCD3 containing the polynucleotide encoding the mutated AAV-binding protein in which AVR5eHD was monomerized and to which PKD3 was further added.

(8) The nucleotide sequence of pET-AVR5eMHCD3 was analyzed in the same manner as in Example 1 (5).

The amino acid sequence of AVR5eMHCD3 is set forth in SEQ ID NO: 76, and the polynucleotide sequence thereof is set forth in SEQ ID NO: 77. In SEQ ID NO: 76, the PelB signal peptide ranges from methionine (Met) at position 1 to alanine (Ala) at position 22, the AAV-binding protein AVR5eMHC (corresponding to the domain ranging from positions 312 to 500 of SEQ ID NO: 1) ranges from serine (Ser) at position 25 to aspartic acid (Asp) at position 213, the AAV extracellular domain 3 (PKD3) (the domain ranging from positions 501 to 597 of SEQ ID NO: 1) ranges from tyrosine (Tyr) at position 214 to lysine (Lys) at position 310, the histidine tag ranges from an amino acid at position 311 to histidine (His) at position 316, and the cysteine tag sequence as a tag for immobilization ranges from cysteine at position 317 to glycine (Gly) at position 323. In SEQ ID NO: 76, methionine of Tyr(Cys)342Met is present at position 55, serine of Gly390Ser is present at position 103, glutamic acid of Lys399Glu is present at position 112, arginine of Ser476Arg is present at position 189, and aspartic acid of Asn487Asp is present at position 200.

### Example 19 Preparation of Monomerized Mutated AAV-Binding Protein (Part 1)

(1) A transformant capable of expressing AVR10s obtained by transforming the *E. coli* BL21 (DE3) strain with pET-AVR10s prepared in Example 17 or a transformant capable of expressing AVR5eMHCD3 obtained by transforming the *E. coli* BL21 (DE3) strain with pET-AVR5eMHCD3 prepared in Example 18 were each inoculated in a 3 mL of2YT liquid medium supplemented with 50 µg/mL kanamycin and aerobically cultured with shaking overnight at 37°C, thereby performing preculture.
(2) The preculture solution of(1) in a volume of 2 mL was inoculated in 200 mL of2YT liquid medium supplemented with 50 µg/mL kanamycin in a 1L baffled flask and aerobically cultured with shaking at 37°C.
(3) The flask was cooled on ice 2.0 hours after the start of culture, IPTG was added to yield a final concentration of 0.1 mM, and shaking culture was continued aerobically overnight at 25°C.
(4) After the end of culture, the cells were recovered by centrifuging the culture solution at 4°C and 8000 rpm for 20 minutes.
(5) The bacterial cells collected in (4) were suspended in a 20 mM Tris-HCl buffer (pH 7.4) containing 150 mM sodium chloride and 20 mM imidazole to yield a concentration of 5 mL/1 g (of cells), and then the cells were disrupted using an ultrasonic generator (Insonator 201M [manufactured by KUBOTA Corporation co., ltd.]) at 8°C and an output power of about 150 W for about 10 minutes. A solution of the disrupted cells was centrifuged twice at 4°C and 8000 rpm for 20 minutes, thereby collecting the supernatant.
(6) The supernatant obtained in (5) was applied to an XK26/20 column column (manufactured by Cytiva) filled with 50 mL of Ni Sepharose 6 Fast Flow (manufactured by Cytiva) which was preliminarily equilibrated with Tris-HCl buffer (pH 7.4) containing 150 mM sodium chloride and 20 mM imidazole (hereinafter, also referred to as "equilibrium solution D"). After washing with the equilibration solution D, elution was performed with 20 mM Tris-HCl buffer (pH 7.4) containing 0.5 M imidazole and 150 mM sodium chloride.
(7) The eluate obtained in (6) was dialyzed against 20 mM Tris-HCl buffer (pH 7.4) containing 150 mM sodium chloride, thereby preparing AVR10s protein or AVR5eMHCD3 protein in the amount required for heat stability evaluation.

### Example 20 Evaluation of Heat Stability of Mutated AAV-Binding Protein (Part 4)

(1) The binding activity of AVR10s prepared in Example 18 to VLP2 was measured using the ELISA method described in Example 2 (6). Based on the absorption at 450 nm corresponding to the above measurement results, the AVR10s solution was diluted with pure water such that the measured values would be the same.
(2) The diluted AVR10s solution was divided into four portions, each portion was mixed with an equal amount of 0.1 M glycine-HCl buffer (pH 3.0). Three of the four samples were heat-treated at 31.7°C, 41.2°C, and 50.0°C, respectively, for 15 minutes, and the remaining one was left to stand still at room temperature (25°C) for 15 minutes. Thereafter, the pH was adjusted to around 6 by mixing each sample with 0.5 M MES buffer (pH 6.0) at a ratio of 3:2, and the binding activity of the protein to VLP2 was measured by the ELISA method described in Example 2 (6).
(3) The residual activity was calculated by dividing the absorbance at 450 nm when the heat treatment described in (2) was performed by the absorbance at 450 nm when standing still was performed at room temperature.
(4) As a comparison target, the heat stability of the wild-type AAV-binding protein (SEQ ID NO: 7) was evaluated in the same manner as in (1) to (3).

Table 17 shows the results. The mutated AAV-binding protein AVR10s prepared in Example 17 had a residual activity higher than that of the wild-type AAV-binding protein regardless of the treatment temperature. This clarifies that AVR10s has significantly improved stability to heat treatment under acidic conditions, i.e., stability to acid and heat, as compared with the wild-type AAV-binding protein.

**[Table 17]**

| AAV-binding protein | | | Residual activity after heat tr eatment [%] | | |
|---|---|---|---|---|---|
| SEQ ID NO | Name | Amino acid substitution | 31.7°C | 41.2°C | 50.0°C |
| 69 | AVR10s | Va1317Asp, Tyr(Cys)342Ser, Lys362Glu, Lys371Asn, Va 1381Ala, Ile382Val, Gly390Ser, Lys399Glu, Ser476Arg, Asn487 Asp | 99 | 79 | 67 |
| 7 | Wild type | | 43 | 35 | 30 |

### Example 21 Evaluation of Heat Stability of Mutated AAV-Binding Protein (Part 5)

(1) The binding activity of AVR5eMHCD3 prepared in Example 18 to VLP2 was measured using the ELISA method described in Example 2 (6). Based on the absorption at 450 nm corresponding to the above measurement results, AVR5eMHCD3 was diluted with pure water such that the measured values would be the same.
(2) The diluted AVR5eMHCD3 solution was divided into four portions, and each portion was diluted 2-fold with pure water. Three of the four samples were heat-treated at 51.7°C, 66.4°C, and 70.0°C, respectively, for 15 minutes, and the remaining one was left to stand still at room temperature (25°C) for 15 minutes. Thereafter, the sample was mixed with 20 mM Tris buffer (pH 7.4) containing 150 mM sodium chloride in a ratio of 3:2, and the binding activity of the protein to VLP2 was measured by the ELISA method described in Example 2 (6).
(3) The residual activity was calculated by dividing the absorbance at 450 nm when the heat treatment described in (2) was performed by the absorbance at 450 nm when standing still was performed at room temperature.
(4) As a comparison target, the heat stability of the wild-type AAV-binding protein (SEQ ID NO: 7) was evaluated in the same manner as in (1) to (3).

Table 18 shows the results. The mutated AAV-binding protein AVR5eMHCD3 prepared in Example 18 had a residual activity higher than that of the wild-type AAV-binding protein regardless of the treatment temperature. This clarifies that AVR5eMHCD3 has significantly improved stability to heat, as compared with the wild-type AAV-binding protein.

**Table 18]**

| AAV-binding protein | | | Residual activity after heat treatm ent [%] | | |
|---|---|---|---|---|---|
| SEQ ID NO | Name | Amino acid substitution | 31.7°C | 41.2°C | 50.0°C |
| 76 | AVR5eMHCD3 | Tyr(Cys)342Met, Gly390Ser, Lys399Glu, Ser476Ar g, Asn487Asp | 96 | 66 | 54 |
| 7 | Wild type | | 72 | 41 | 34 |

### Example 22 Preparation of AAV-Binding Protein-Immobilized Gel (Part 2)

(1) Wild-type AAV-binding protein-immobilized gel and AVR5eHC-immobilized gel were prepared in the same manner as in Example 12. Specifically, to 1 mL gel into which a maleimide group was introduced, 10 mg of AVR5eHC prepared in Example 10 or the wild-type AAV-binding protein and TCEP with a final concentration of 0.1 mM as a reducing agent were added. The reaction was carried out by shaking at pH 7.4 and 4°C for 15 hours.
(2) The amount of each protein immobilized on gel was estimated by measuring the filtrate after immobilization in (1) with an absorptiometer and calculating the amount of non-immobilized protein. AVR5eHC was immobilized at 9 mg/mL (gel) on the AVR5eHC-immobilized gel, and the wild-type AAV-binding protein was immobilized at 10 mg/mL (gel) on the wild-type AAV-binding protein-immobilized gel.
(3) Each immobilized gel prepared in (1) was placed in an amount of 0.065 mL in Cosmo Spin Filter G (manufactured by Nacalai Tesque), thereby preparing a mini column (named AVR5eHC-immobilized gel mini column and wild-type AAV-binding protein-immobilized gel mini column, respectively). Each mini column was equilibrated with 50 mM phosphate buffer (pH 7.4) containing 150 mM sodium chloride and 0.05% (w/v) Tween 20 (trade name) (hereinafter also referred to as "solution A"), and 2.9 × 10¹⁰ VG (number of vector genomes) of the AAV2-EGFP purified product obtained in Example 11 was applied thereto.
(4) Shaking was performed at 1600 rpm for 5 minutes using an incubator shaker MBR-022UP (manufactured by TAITEC CORPORATION), and then the filtrate was collected by centrifugation at 1000 G for 2 minutes. The solution A was added in an amount of 200 µL to each mini column again, and the mixture was shaken for 1 minute and then centrifuged to recover 400 µL together with the above filtrate (hereinafter, also referred to as "flow through").
(5) Each column was washed two times with 400 µL of the solution A, and each filtrate was collected (the filtrates collected in the first and second times are also referred to as "wash 1" and "wash 2," respectively). To each mini column, 200 µL of 50 mM phosphate buffer (pH 2.5) containing 150 mM sodium chloride and 0.05% (w / v) Tween 20 (trade name) (hereinafter, also referred to as "solution B") was added. Shaking was performed for 5 minutes, and then the eluate was collected by centrifugation. The solution B was added in an amount of 200 µL to each mini column again, and the mixture was shaken for 1 minute and then centrifuged to recover 400 µL together with the above eluate (hereinafter, also referred to as "eluate 1").
(6) Further, 400 µL of the solution B was added, and the mixture was centrifuged to collect the eluate (hereinafter, also referred to as "eluate 2"). The eluates 1 and 2 were neutralized by adding 100 µL of 1 M Tris-HCl buffer (pH 8.5) containing 20 mM magnesium chloride.
(7) The numbers of AAV2-EGFP vector genomes contained in the flow through obtained in (3), wash 1, wash 2, and eluate 1 obtained in (5), and eluate 2 obtained in (6) were quantified by qPCR using AAVpro Titration Kits (manufactured by Takara Bio Inc.).

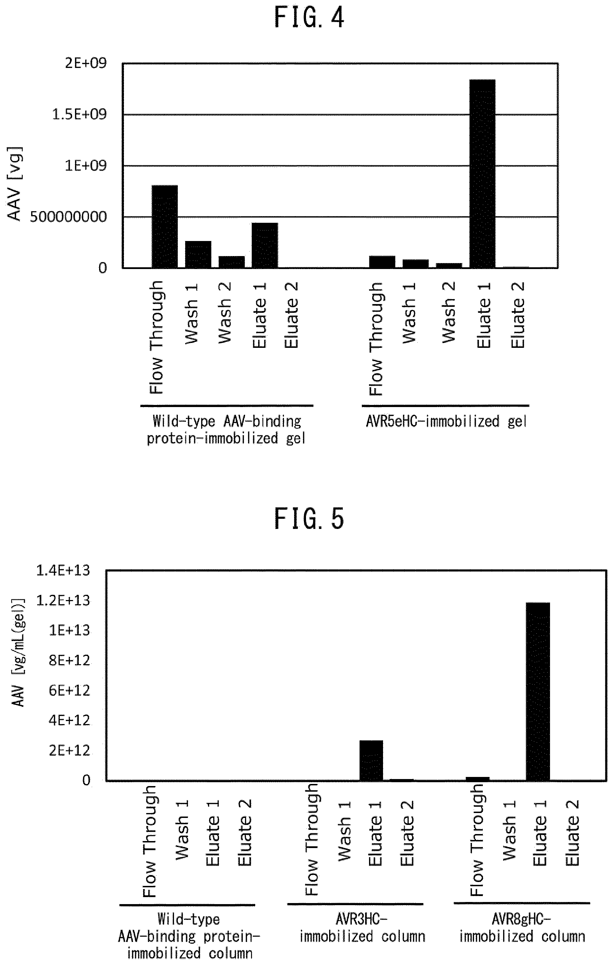

FIG. 4 shows the results. In the wild-type AAV-binding protein-immobilized gel mini column, most of the applied AAV was confirmed in the flow through fraction. Meanwhile, in the AVR5eHC-immobilized gel mini column, almost all of the applied AAV was adsorbed on the gel, and elution from the gel was confirmed in the eluate 1 fraction. The above results clarify that AAV can be purified with higher efficiency by an AAV adsorbent containing gel and the AAV-binding protein of the invention immobilized on the gel than when wild-type AVR is used.

### Example 23 Preparation of AAV-Binding Protein-Immobilized Gel (Part 3)

(1) By the same method as in Example 9, pET-AVR3 and pET-AVR8g were used as templates, thereby obtaining pET-AVR3HC and pET-AVR8gHC. Further, AVR3HC (protein having an immobilization tag at the C-terminus of AVR3) and AVR8gHC (protein having an immobilization tag at the C-terminus of AVR8g) were prepared in the same manner as in Example 10. AVR3HC-immobilized gel and AVR8gHC-immobilized gel were prepared using the prepared proteins in the manner as in Example 22. In addition, wild-type AAV-binding protein-immobilized gel was prepared in the same manner as in Example 22.
(2) The amount of each protein immobilized on gel was estimated by measuring the filtrate after immobilization in (1) with an absorptiometer and calculating the amount of non-immobilized protein. AVR3HC was immobilized at 3.8 mg/mL (gel) on the AVR3HC-immobilized gel, and AVR8gHC was immobilized at 5.7 mg/mL (gel) on the AVR8gHC-immobilized gel.
(3) Cosmo Spin Filter G (manufactured by Nacalai Tesque) was filled with 0.065 mL of the wild-type AAV-binding protein-immobilized gel, 0.065 mL of the AVR3HC-immobilized gel, and 0.018 mL of the AVR8gHC-immobilized gel prepared in (1), respectively, thereby preparing mini columns (named wild-type AAV-binding protein-immobilized gel mini column, AVR3HC-immobilized gel mini column, and AVR8gHC-immobilized gel mini column, respectively). Each mini column was equilibrated with 50 mM acetate buffer (pH 6.0) containing 150 mM sodium chloride (hereinafter also referred to as "solution A"). Subsequently, the AAV2-EGFP purified product obtained in Example 11 was applied to the flow through fraction in a plurality of times until it was confirmed that AAV2-EGFP was sufficiently leaked. Eventually, the amount of applied AAV2-EGFP per column was 2.9 × 10¹⁰ VG for the wild-type AAV-binding protein-immobilized gel mini column, 3.9 × 10¹⁰ VG for the AVR3HC-immobilized gel mini column, and 2.4 × 10¹¹ VG for the AVR8gHC-immobilized gel mini column.
(4) To elute and recover AAV2-EGFP bound to the gel, each column was washed two times with 200 µL of the solution A, and each filtrate was recovered (hereinafter, also referred to as "wash 1"). To each mini column, 200 µL of 50 mM phosphate buffer (pH 3.0) containing 150 mM sodium chloride (hereinafter, also referred to as "solution B") was added. Shaking was performed for 5 minutes, and then the eluate was collected by centrifugation. The solution B was added in an amount of 200 µL to each mini column again, and the mixture was shaken for 1 minute and then centrifuged to recover 400 µL together with the above eluate (hereinafter, also referred to as "eluate 1").
(5) Further, 400 µL of the solution B was added, and the mixture was centrifuged to collect the eluate (hereinafter, also referred to as "eluate 2"). The eluates 1 and 2 were neutralized by adding 100 µL of 1 M Tris-HCl buffer (pH 8.5) containing 20 mM magnesium chloride.
(6) The numbers of AAV2-EGFP vector genomes contained in the flow through, wash 1, eluate 1, and eluate 2 obtained in (3) to (5) were quantified by qPCR using AAVpro Titration Kits (manufactured by Takara Bio Inc.).

FIG. 5 shows the results. It was found that most of the AAV2-EGFP bound to the eluate1 fraction was eluted and recovered. From the amount of AAV2-EGFP recovered by elution, the amount of AAV2-EGFP bound to each mini column was converted per 1 mL of protein-immobilized gel. As a result, it was 8.6 × 10⁹ VG/mL (gel) for wild-type AAV-binding protein-immobilized gel, 2.8 × 10¹² VG/mL (gel) for AVR3HC-immobilized gel, and 1.2 × 10¹³ VG/mL

## Claims

1. An adeno-associated virus (AAV)-binding protein which is selected from any one of the following (i) to (iii):
(i) an AAV-binding protein comprising at least amino acid residues from serine at position 312 to aspartic acid at position 500 of an amino acid sequence set forth in SEQ ID NO: 1, wherein at least any one of the following amino acid substitutions (1) to (137) is present at the amino acid residues from positions 312 to 500:
(1) a substitution of glycine with serine at position 390 of SEQ ID NO: 1,
(2) a substitution of isoleucine with phenylalanine at position 319 of SEQ ID NO: 1,
(3) a substitution of leucine with proline at position 321 of SEQ ID NO: 1,
(4) a substitution of proline with leucine at position 322 of SEQ ID NO: 1,
(5) a substitution of asparagine with aspartic acid or serine at position 324 of SEQ ID NO: 1,
(6) a substitution of glutamine with arginine at position 327 of SEQ ID NO: 1,
(7) a substitution of asparagine with aspartic acid or tyrosine at position 329 of SEQ ID NO: 1,
(8) a substitution of alanine with glycine at position 330 of SEQ ID NO: 1,
(9) a substitution of tyrosine with cysteine at position 331 of SEQ ID NO: 1,
(10) a substitution of valine with glutamine at position 332 of SEQ ID NO: 1,
(11) a substitution of leucine with valine or proline at position 333 of SEQ ID NO: 1,
(12) a substitution of glutamine with arginine at position 334 of SEQ ID NO: 1,
(13) a substitution of proline with leucine at position 337 of SEQ ID NO: 1,
(14) a substitution of lysine with arginine or glutamic acid at position 338 of SEQ ID NO: 1,
(15) a substitution of glutamic acid with glycine at position 340 of SEQ ID NO: 1,
(16) a substitution of threonine with alanine at position 341 of SEQ ID NO: 1,
(17) a substitution of tyrosine with cysteine, histidine, or asparagine at position 342 of SEQ ID NO: 1,
(18) a substitution of threonine with methionine at position 343 of SEQ ID NO: 1,
(19) a substitution of tyrosine with histidine at position 344 of SEQ ID NO: 1,
(20) a substitution of aspartic acid with asparagine at position 345 of SEQ ID NO: 1,
(21) a substitution of tryptophan with leucine or arginine at position 346 of SEQ ID NO: 1,
(22) a substitution of glutamine with leucine or proline at position 347 of SEQ ID NO: 1,
(23) a substitution of leucine with proline at position 348 of SEQ ID NO: 1,
(24) a substitution of isoleucine with threonine at position 349 of SEQ ID NO: 1,
(25) a substitution of threonine with methionine at position 350 of SEQ ID NO: 1,
(26) a substitution of histidine with leucine at position 351 of SEQ ID NO: 1,
(27) a substitution of proline with leucine at position 352 of SEQ ID NO: 1,
(28) a substitution of arginine with cysteine at position 353 of SEQ ID NO: 1,
(29) a substitution of aspartic acid with glycine at position 354 of SEQ ID NO: 1,
(30) a substitution of tyrosine with histidine, asparagine, or cysteine at position 355 of SEQ ID NO: 1,
(31) a substitution of serine with cysteine at position 356 of SEQ ID NO: 1,
(32) a substitution of glycine with cysteine at position 357 of SEQ ID NO: 1,
(33) a substitution of histidine with arginine or leucine at position 363 of SEQ ID NO: 1,
(34) a substitution of serine with proline at position 364 of SEQ ID NO: 1,
(35) a substitution of glutamine with arginine at position 365 of SEQ ID NO: 1,
(36) a substitution of isoleucine with threonine at position 366 of SEQ ID NO: 1,
(37) a substitution of leucine with proline at position 367 of SEQ ID NO: 1,
(38) a substitution of lysine with arginine at position 368 of SEQ ID NO: 1,
(39) a substitution of leucine with glutamine or proline at position 369 of SEQ ID NO: 1,
(40) a substitution of serine with alanine at position 370 of SEQ ID NO: 1,
(41) a substitution of lysine with glutamic acid at position 371 of SEQ ID NO: 1,
(42) a substitution of threonine with alanine at position 373 of SEQ ID NO: 1,
(43) a substitution of leucine with proline at position 376 of SEQ ID NO: 1,
(44) a substitution of tyrosine with cysteine at position 377 of SEQ ID NO: 1,
(45) a substitution of phenylalanine with serine at position 379 of SEQ ID NO: 1,
(46) a substitution of valine with alanine at position 381 of SEQ ID NO: 1,
(47) a substitution of glutamic acid with glycine at position 384 of SEQ ID NO: 1,
(48) a substitution of asparagine with serine at position 387 of SEQ ID NO: 1,
(49) a substitution of histidine with glutamine, leucine, or arginine at position 389 of SEQ ID NO: 1,
(50) a substitution of glutamic acid with lysine at position 391 of SEQ ID NO: 1,
(51) a substitution of tyrosine with cysteine at position 393 of SEQ ID NO: 1,
(52) a substitution of valine with alanine at position 396 of SEQ ID NO: 1,
(53) a substitution of lysine with glutamic acid or arginine at position 399 of SEQ ID NO: 1,
(54) a substitution of arginine with serine at position 406 of SEQ ID NO: 1,
(55) a substitution of valine with alanine at position 412 of SEQ ID NO: 1,
(56) a substitution of glutamine with leucine at position 415 of SEQ ID NO: 1,
(57) a substitution of phenylalanine with serine at position 416 of SEQ ID NO: 1,
(58) a substitution of glutamine with leucine at position 441 of SEQ ID NO: 1,
(59) a substitution of tyrosine with phenylalanine at position 442 of SEQ ID NO: 1,
(60) a substitution of lysine with arginine at position 448 of SEQ ID NO: 1,
(61) a substitution of glutamic acid with glycine at position 453 of SEQ ID NO: 1,
(62) a substitution of lysine with arginine at position 455 of SEQ ID NO: 1,
(63) a substitution of glutamic acid with glycine at position 458 of SEQ ID NO: 1,
(64) a substitution of alanine with serine at position 461 of SEQ ID NO: 1,
(65) a substitution of serine with arginine at position 476 of SEQ ID NO: 1,
(66) a substitution of leucine with proline at position 477 of SEQ ID NO: 1,
(67) a substitution of asparagine with aspartic acid at position 487 of SEQ ID NO: 1,
(68) a substitution of asparagine with aspartic acid at position 492 of SEQ ID NO: 1,
(69) a substitution of isoleucine with asparagine or serine at position 319 of SEQ ID NO: 1,
(70) a substitution of threonine with isoleucine at position 320 of SEQ ID NO: 1,
(71) a substitution of lysine with glutamic acid at position 323 of SEQ ID NO: 1,
(72) a substitution of leucine with glutamine or proline at position 328 of SEQ ID NO: 1,
(73) a substitution of tyrosine with histidine at position 331 of SEQ ID NO: 1,
(74) a substitution of valine with alanine or glutamic acid at position 332 of SEQ ID NO: 1,
(75) a substitution of proline with glutamine at position 336 of SEQ ID NO: 1,
(76) a substitution of proline with glutamine at position 337 of SEQ ID NO: 1,
(77) a substitution of lysine with asparagine at position 338 of SEQ ID NO: 1,
(78) a substitution of tyrosine with arginine at position 342 of SEQ ID NO: 1,
(79) a substitution of threonine with serine at position 350 of SEQ ID NO: 1,
(80) a substitution of isoleucine with phenylalanine at position 366 of SEQ ID NO: 1,
(81) a substitution of valine with alanine at position 383 of SEQ ID NO: 1,
(82) a substitution of glutamine with arginine at position 386 of SEQ ID NO: 1,
(83) a substitution of histidine with aspartic acid at position 389 of SEQ ID NO: 1,
(84) a substitution of glycine with cysteine at position 392 of SEQ ID NO: 1,
(85) a substitution of valine with alanine at position 394 of SEQ ID NO: 1,
(86) a substitution of isoleucine with valine at position 409 of SEQ ID NO: 1,
(87) a substitution of serine with glycine at position 476 of SEQ ID NO: 1,
(88) a substitution of threonine with serine at position 490 of SEQ ID NO: 1,
(89) a substitution of serine with proline at position 312 of SEQ ID NO: 1,
(90) a substitution of alanine with serine at position 313 of SEQ ID NO: 1,
(91) a substitution of valine with aspartic acid at position 317 of SEQ ID NO: 1,
(92) a substitution of glutamine with proline at position 318 of SEQ ID NO: 1,
(93) a substitution of threonine with alanine at position 320 of SEQ ID NO: 1,
(94) a substitution of lysine with arginine at position 323 of SEQ ID NO: 1,
(95) a substitution of valine with alanine or glutamic acid at position 326 of SEQ ID NO: 1,
(96) a substitution of glutamine with histidine or leucine at position 327 of SEQ ID NO: 1,
(97) a substitution of asparagine with histidine or isoleucine at position 329 of SEQ ID NO: 1,
(98) a substitution of valine with alanine at position 332 of SEQ ID NO: 1,
(99) a substitution of glutamic acid with glycine or valine at position 335 of SEQ ID NO: 1,
(100) a substitution of glutamic acid with valine at position 340 of SEQ ID NO: 1,
(101) a substitution of threonine with proline at position 341 of SEQ ID NO: 1,
(102) a substitution of threonine with serine at position 343 of SEQ ID NO: 1,
(103) a substitution of tyrosine with phenylalanine at position 344 of SEQ ID NO: 1,
(104) a substitution of tryptophan with cysteine at position 346 of SEQ ID NO: 1,
(105) a substitution of methionine with leucine at position 359 of SEQ ID NO: 1,
(106) a substitution of glutamic acid with lysine or valine at position 360 of SEQ ID NO: 1,
(107) a substitution of glycine with cysteine at position 361 of SEQ ID NO: 1,
(108) a substitution of lysine with glutamic acid, asparagine, or glycine at position 362 of SEQ ID NO: 1,
(109) a substitution of serine with leucine at position 364 of SEQ ID NO: 1,
(110) a substitution of lysine with asparagine or aspartic acid at position 371 of SEQ ID NO: 1,
(111) a substitution of leucine with proline or glutamine at position 372 of SEQ ID NO: 1,
(112) a substitution of proline with leucine at position 374 of SEQ ID NO: 1,
(113) a substitution of glutamic acid with glycine or valine at position 378 of SEQ ID NO: 1,
(114) a substitution of phenylalanine with tyrosine or cysteine at position 379 of SEQ ID NO: 1,
(115) a substitution of lysine with glutamic acid at position 380 of SEQ ID NO: 1,
(116) a substitution of valine with aspartic acid at position 381 of SEQ ID NO: 1,
(117) a substitution of isoleucine with valine at position 382 of SEQ ID NO: 1,
(118) a substitution of glutamic acid with valine at position 384 of SEQ ID NO: 1,
(119) a substitution of valine with aspartic acid or isoleucine at position 394 of SEQ ID NO: 1,
(120) a substitution of asparagine with serine at position 395 of SEQ ID NO: 1,
(121) a substitution of threonine with serine at position 397 of SEQ ID NO: 1,
(122) a substitution of glutamic acid with valine at position 401 of SEQ ID NO: 1,
(123) a substitution of arginine with histidine at position 403 of SEQ ID NO: 1,
(124) a substitution of arginine with histidine at position 406 of SEQ ID NO: 1,
(125) a substitution of glutamine with arginine at position 415 of SEQ ID NO: 1,
(126) a substitution of threonine with alanine at position 426 of SEQ ID NO: 1,
(127) a substitution of glutamine with leucine or arginine at position 432 of SEQ ID NO: 1,
(128) a substitution of glutamine with arginine at position 441 of SEQ ID NO: 1,
(129) a substitution of histidine with leucine at position 443 of SEQ ID NO: 1,
(130) a substitution of lysine with glutamic acid at position 448 of SEQ ID NO: 1,
(131) a substitution of isoleucine with valine at position 456 of SEQ ID NO: 1,
(132) a substitution of aspartic acid with asparagine at position 483 of SEQ ID NO: 1,
(133) a substitution of serine with leucine at position 488 of SEQ ID NO: 1,
(134) a substitution of valine with glutamic acid or isoleucine at position 499 of SEQ ID NO: 1;
(ii) an AAV-binding protein comprising at least amino acid residues from serine at position 312 to aspartic acid at position 500 of the amino acid sequence set forth in SEQ ID NO: 1, wherein at least any one of the amino acid substitutions (1) to (134) is present at the amino acid residues from positions 312 to 500, wherein one or more of substitutions, deletions, insertions, and additions of one or several amino acid residues are further present at one or several positions other than the amino acid substitutions shown in (1) to (134), and wherein the AAV-binding protein has AAV-binding activity; and
(iii) an AAV-binding protein comprising an amino acid sequence, wherein the amino acid sequence has 70% or more homology to an entire amino acid sequence in which at least any one of the amino acid substitutions (1) to (134) is present in an amino acid sequence ranging from serine at position 312 to aspartic acid at position 500 of the amino acid sequence set forth in SEQ ID NO: 1, wherein the at least any one of the amino acid substitutions remains in the amino acid sequence, and wherein the AAV-binding protein has AAV-binding activity.

2. The AAV-binding protein according to claim 1, which is selected from any one of the following (iv) to (vi):
(iv) an AAV-binding protein comprising at least amino acid residues from serine at position 312 to aspartic acid at position 500 of the amino acid sequence set forth in SEQ ID NO: 1, wherein at least the following amino acid substitution (1) is present at the amino acid residues from positions 312 to 500:
(1) a substitution of glycine with serine at position 390 of SEQ ID NO: 1,
(v) an AAV-binding protein comprising at least amino acid residues from serine at position 312 to aspartic acid at position 500 of the amino acid sequence set forth in SEQ ID NO: 1, wherein at least the amino acid substitution (1) is present at the amino acid residues from positions 312 to 500, wherein one or more of substitutions, deletions, insertions, and additions of one or several amino acid residues are further present at one or several positions other than the amino acid substitution shown in (1), and wherein the AAV-binding protein has AAV-binding activity; and
(vi) an AAV-binding protein comprising an amino acid sequence, wherein the amino acid sequence has 70% or more homology to an entire amino acid sequence in which at least the amino acid substitution (1) is present in an amino acid sequence ranging from serine at position 312 to aspartic acid at position 500 of the amino acid sequence set forth in SEQ ID NO: 1, wherein the amino acid substitution (1) remains in the amino acid sequence, and wherein the AAV-binding protein has AAV-binding activity.

3. The AAV-binding protein according to claim 2, wherein at least any one of amino acid substitutions shown in the following (A) to (K) is present:
(A) a substitution of glycine with serine at position 390 of SEQ ID NO: 1 and a substitution of tyrosine with cysteine at position 342 of SEQ ID NO: 1,
(B) a substitution of glycine with serine at position 390 of SEQ ID NO: 1, a substitution of tyrosine with cysteine at position 342 of SEQ ID NO: 1, and a substitution of serine with arginine at position 476 of SEQ ID NO: 1,
(C) a substitution of glycine with serine at position 390 of SEQ ID NO: 1, a substitution of isoleucine with phenylalanine at position 319 of SEQ ID NO: 1, a substitution of tyrosine with cysteine at position 342 of SEQ ID NO: 1, and a substitution of serine with arginine at position 476 of SEQ ID NO: 1,
(D) a substitution of glycine with serine at position 390 of SEQ ID NO: 1, a substitution of tyrosine with cysteine at position 342 of SEQ ID NO: 1, a substitution of serine with arginine at position 476 of SEQ ID NO: 1, and a substitution of asparagine with aspartic acid at position 487 of SEQ ID NO: 1,
(E) a substitution of glycine with serine at position 390 of SEQ ID NO: 1, a substitution of isoleucine with phenylalanine at position 319 of SEQ ID NO: 1, a substitution of lysine with glutamic acid at position 323 of SEQ ID NO: 1, a substitution of tyrosine with cysteine at position 342 of SEQ ID NO: 1, and a substitution of serine with arginine at position 476 of SEQ ID NO: 1,
(F) a substitution of glycine with serine at position 390 of SEQ ID NO: 1, a substitution of tyrosine with cysteine at position 342 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
(G) a substitution of glycine with serine at position 390 of SEQ ID NO: 1, a substitution of asparagine with aspartic acid at position 324 of SEQ ID NO: 1, a substitution of tyrosine with cysteine at position 342 of SEQ ID NO: 1, a substitution of lysine with glutamic acid at position 362 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
(H) a substitution of glycine with serine at position 390 of SEQ ID NO: 1, a substitution of valine with aspartic acid at position 317 of SEQ ID NO: 1, a substitution of tyrosine with cysteine at position 342 of SEQ ID NO: 1, a substitution of lysine with asparagine at position 371 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
(I) a substitution of glycine with serine at position 390 of SEQ ID NO: 1, a substitution of valine with aspartic acid at position 317 of SEQ ID NO: 1, a substitution of asparagine with aspartic acid at position 324 of SEQ ID NO: 1, a substitution of tyrosine with cysteine at position 342 of SEQ ID NO: 1, a substitution of lysine with glutamic acid at position 362 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1,
(J) a substitution of glycine with serine at position 390 of SEQ ID NO: 1, a substitution of valine with aspartic acid at position 317 of SEQ ID NO: 1, a substitution of tyrosine with cysteine at position 342 of SEQ ID NO: 1, a substitution of lysine with glutamic acid at position 362 of SEQ ID NO: 1, a substitution of lysine with asparagine at position 371 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1, and
(K) a substitution of glycine with serine at position 390 of SEQ ID NO: 1, a substitution of valine with aspartic acid at position 317 of SEQ ID NO: 1, a substitution of tyrosine with serine at position 342 of SEQ ID NO: 1, a substitution of lysine with glutamic acid at position 362 of SEQ ID NO: 1, a substitution of lysine with asparagine at position 371 of SEQ ID NO: 1, a substitution of lysine with alanine at position 381 of SEQ ID NO: 1, a substitution of isoleucine with valine at position 382 of SEQ ID NO: 1, a substitution of glycine with serine at position 390 of SEQ ID NO: 1, a substitution of lysine with glutamic acid at position 399 of SEQ ID NO: 1, a substitution of serine with arginine at position 476 of SEQ ID NO: 1, and a substitution of asparagine with aspartic acid at position 487 of SEQ ID NO: 1.

4. The AAV-binding protein according to claim 3, which is selected from any one of the following (vii) to (ix):
(vii) an AAV-binding protein comprising at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76;
(viii) an AAV-binding protein comprising at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76, wherein one or more of substitutions, deletions, insertions, and additions of one or several amino acid residues are further present at one or several positions of the amino acid residues from positions 25 to 213 other than the amino acid substitution in the amino acid sequence, and wherein the AAV-binding protein has AAV-binding activity; and
(ix) an AAV-binding protein comprising amino acid residues having 70% or more homology to at least amino acid residues from serine at position 25 to aspartic acid at position 213 of an amino acid sequence set forth in any one of SEQ ID NOS: 18, 32, 34, 38, 42, 46, 53, 55, 59, 63, 69, and 76, wherein one or more amino acid substitutions of said amino acid sequence are present, and having AAV-binding activity.

5. A polynucleotide encoding the AAV-binding protein according to any one of claims 1 to 4.

6. An expression vector comprising the polynucleotide according to claim 5.

7. A transformant obtained by transforming a host with the expression vector according to claim 6.

8. The transformant according to claim 7, wherein the host is *Escherichia coli.*

9. A method for producing an AAV-binding protein, comprising a step of allowing the AAV-binding protein to be expressed by culturing the transformant according to claim 7 or 8 and a step of recovering the AAV-binding protein expressed from the obtained culture.

10. An AAV adsorbent comprising an insoluble carrier and the AAV-binding protein according to any one of claims 1 to 4 which is immobilized on the carrier.

11. A column containing the AAV adsorbent according to claim 10.

12. A method for purifying AAV, comprising a step of adding a solution containing AAV to the column according to claim 11 to allow the AAV to be adsorbed by the adsorbent and a step of eluting the AAV adsorbed by the adsorbent using an eluate.
